Europäisches Patentamt

(19) European Patent Office

Office européen des brevets

(11) Publication number: **0 308 265**
A2

# (12) EUROPEAN PATENT APPLICATION

(21) Application number: 88308625.8

(22) Date of filing: 16.09.88

(51) Int. Cl.⁴: **G 01 N 33/574**
// A61K39/00, C12N15/00,
C12N5/00, C12P21/00, C07K3/20

(30) Priority: 16.09.87 US 97910   11.12.87 US 131815

(43) Date of publication of application:
22.03.89 Bulletin 89/12

(84) Designated Contracting States: ES

(71) Applicant: INTERNATIONAL GENETIC ENGINEERING, INC.
1545 - 17th Street
Santa Monica California 90404 (US)

(72) Inventor: Fareed, George C.
721 Thayer Avenue
Los Angeles California (US)

Sen, Arup
3430 Jasmine, Apt. 202
Los Angeles California (US)

Ghosh-Dastidar, Pradip
3556 South Barrington Avenue
Los Angeles California (US)

Liu, Alvin
807 Fifth St., Apt. 6
Santa Monica California (US)

Lee, Jar-How
2840 Glendon Avenue
Los Angeles California (US)

(74) Representative: Brown, John David et al
FORRESTER & BOEHMERT Widenmayerstrasse 4/I
D-8000 München 22 (DE)

(54) Methods and compositions relating to regression-associated antigens.

(57) Regression associated antigens (RAAs) are identified in material from neoplastic cells by their immunological reactivity with regression associated antibodies from the serum of patients diagnosed as undergoing regression of a tumor. Regression associated antibodies (RAAbs) are identified by their absence during progression of a neoplastic disease state and by their presence in a diagnosed state of regression. The antigens are purified, used to monitor the condition of cancer patients, and production of antibodies and treatments employing those antibodies are described. It is also disclosed that RAAs are expressed by M. hyorhinis and may be expressed by expression of provided nucleotide sequences in recombinant host cells. RAAs and nucleic acids encoding RAAs (or portions thereof) and RAAbs may be used in diagnostic assays and immunotherapy. RAAbs and fragments portion thereof may be used in passive immunization therapy and radioisotope or magnetic resonance scanning. A monoclonal RAAb and a hybrdoma secreting it are also provided.

RAAs DETECTED IN MEMBRANES OR MEDIA AFTER
I.L.(Pt-1) OR S.C.(Pt-2, rabbit 209) IMMUNIZATIONS

FIG.1

EP 0 308 265 A2

**Description**

## METHODS AND COMPOSITIONS RELATING TO REGRESSION-ASSOCIATED ANTIGENS

This application claims priority based on PCT Application Serial No. PCT/US87/00334, filed February 18, 1987, and is a continuation-in-part of Application Serial No. 097,910, filed September 16, 1987, which is a continuation-in-part of Application Serial No. 837,494 filed March 7, 1986.

## Background

The present invention pertains in general to antigens associated with tumors and uses therefor, and in particular to regression-associated antigens (RAAs), to preparations containing RAAs and to uses for such antigens and preparations.

One approach to diagnosis and treatment of cancers involves the development of polyclonal and monoclonal antibodies against tumor-associated antigens. In almost all reported cases, the immunogens used to obtain antibodies directed against tumor cell components are intact tumor cells or are membrane proteins obtained from the cells.

Early work in this field involved the identification of onco-fetal antigens and blood group antigens [Springer, Science, 224, 1198 (1984)] which are expressed by malignant cells and shed into the bloodstream in some instances. Antigens associated with tumor cells may be identified by immunoblotting methods. Du Bois et al., J. Immunol. Methods, 63, 7 (1983).

In particular, monoclonal antibodies reactive with the surface of human breast carcinoma cells may be generated and characterized using membrane-enriched fractions of metastatic carcinoma lesions. Schlom et al., Cancer, 54 (11 suppl.), 2777-2794 (1984). One monoclonal antibody reacts with a 220,000 to 400,000 dalton high molecular weight glycoprotein complex found in 50% of human mammary carcinomas and 80% of human colon carcinomas. Scholm et al., supra. Mouse monoclonal antibody L6 recognizes a ganglioside antigen that is of particular interest because it is expressed at the surface of cells from most human carcinomas of lung, breast, colon and ovary, while it is present in only trace amounts at the surface of normal cells. Hellstrom et al., Cancer Res., 46, 3917-3923 (1986).

A number of other monoclonal antibodies reactive with tumor-associated antigens on the surfaces of other human cancer cells, including ovarian, pancreatic and intestinal malignancies, may thus be obtained. Proceedings of the UCLA Symposium on Monoclonal Antibodies and Cancer Therapy, Reisfeld et al., eds., Alan R. Liss, Inc., New York, 3-74, 97-109 and 149-164 (1985).

Monoclonal and polyclonal anti-tumor cell antibodies described to date are directed against determinants of human tumor cell antigens which may elicit an immune response in test animals chosen for the production of tumor-specific antibodies. It is not known whether patients harboring tumors or treated with specific and/or non-specific immune stimulants produce antibodies against these antigenic determinants. Therefore, the relevance of such antibodies in mediating regression of tumors in patients is unclear. Passive transfer of such antibodies generated in animals into patients has met with limited success. Lowder et al., Western J. Med., 143, 810 (1985).

A clinical approach toward active immunotherapy of tumors involves a generalized stimulation of the patient's own immune system using non-specific stimulants such as components of the walls of two bacterial cells, Mycobacterium bovis (BCG strain) and Corynebacterium parvum or "detoxified" bacterial endotoxin. In parallel, biological response modifiers such as interleukin-2 may be used to induce activation of the immune system and cause tumor cell destruction. Mule et al., J. Immunol., 135, 646 (1985); and Rosenberg et al., New Engl. J. Med., 313, 1485 (1985).

In an approach called active specific immunotherapy, immunization of cancer patients may be attempted with preparations derived from allogeneic tumor cells (tumor cells obtained from a histopathologically similar tumor of a different patient). This may specifically stimulate the patient's own immune system to possibly unique antigenic structures present on a particular malignant, cell type, and may thus induce tumor regression. Lachman et al. Br. J. Cancer, 51, 415-417 (1985); and Wallack et al., Surgery, 96, 791-800 (1984). Active specific immunotherapy may also be attempted by systematically injecting autologous (autochthonous) tumor cells (i.e., cells derived from the tumor mass of the same patient) intradermally or subcutaneously. Laucius et al., Cancer, 40, 2091 (1977).

Various preparations of autologous tumor cells or of allogeneic tumor cell lines have been used in active specific immunotherapy. Key et al., Adv. Immun. Cancer Ther., 1, 195-219 (1985); Weisenburger et al., J. Biol. Response Mod., 1, 57-66 (1982); and Kan-Mitchell et al., Proceedings of the UCLA Symposium on Monoclonal Antibodies and Cancer Therapy, supra, 523-536. The preparations are generally treated with irradiation, mechanical disruption, or freeze-thaw cycles to render the tumor cells non-viable. They are then used as immunogens, with or without an adjuvant, and are administered by a variety of routes (such as intradermal, subcutaneous, intramuscular or intralymphatic) for the purpose of immunizing cancer patients. Relatively little toxicity has been reported with these preparations, and encouraging clinical responses have been obtained in significant numbers of advanced cancer patients.

One major limitation of attempts at active specific immunotherapy is the undefined nature of the tumor cell preparations (generally intact irradiated cell suspensions or mechanically disrupted lysates of cells). Cells from autologous tumor cells grown in tissue culture or continuously passaged tumor cell lines may undergo significant changes in their phenotypes during growth in laboratory culture. Reagents or tests to standardize the preparations for their expected potency have not been available. Membrane proteins may be shed from intact irradiated cells and proteins in cell lysates may be degraded by proteolytic enzymes. Different preparations used in attempts at active specific immunotherapy may, therefore, have variable efficacies although similar processes and cell types are utilized. Furthermore, the means of monitoring the immune response of individual patients is not available for tailoring the immunization dose and the immunization schedule for optimal clinical outcome. In addition, it is often the case that autologous tumor cell preparations are not practical because of a lack of adequate tumor from the patient to be treated.

Results from a number of animal models support the use of tumor cell components in active specific immunization to induce tumor regression [Key et al., J. Biol. Response Mod., 3, 359-365 (1984); and Srivastava et al., Proc. Nat'l Acad. Sci. (USA), 83, 3407-3411 (1986)]. Neoplasms induced in mice by polycyclic aromatic hydrocarbons such as 3-methylcholoanthrene express individually distinct tumor-associated transplantation antigens. These antigens are immunogenic in their syngeneic hosts and provide transplantation immunity only against their respective tumors and not against independent tumors induced by the same or a different carcinogen or against tumors of viral origin. Transplantation immunity in mice may be elicited by prior growth and removal of tumor transplants or by immunization with irradiated tumor cells, tumor cell membranes or solubilized antigen preparations.

The results of clinical studies with autologous tumor cell vaccines are encouraging when a potent adjuvant such as BCG is used along with the tumor cell suspension. Hoover et al., Cancer Res., 44, 1671-1676 (1984). Some patients immunized through different routes with allogeneic cells with or without adjuvants have shown significant, often dramatic, clinical responses. Weisenburger, J. Biol. Response Mod., 1, 57-66 (1982); Mitchell, in Proceedings of the UCLA Symposium on Monoclonal Antibodies and Cancer Therapy, supra, 495-504. Certain key antigen components of tumor cells may be able to to elicit protective/regressor antibodies in humans.

A knowledge of the antibody response associated with human tumor regression following active specific immunotherapy and identification of subcellular components involved in eliciting such specific antibodies should lead to the development of improved active specific immunogens for cancer immunotherapy. Thus, it is desirable to develop: (i) preparations which will be more enriched in the relevant specific immunogens; (ii) reagents to screen better cell sources and quantitate the immunogens in preparations derived from these cells such that different preparations can be meaningfully standardized; and (iii) assay methods to monitor patients' specific immune response to these immunogens, thereby providing the physician an ability to adjust the treatment protocol in order to produce a better clinical outcome.

A monoclonal antibody designated PF/2A is a product of standard monoclonal antibody production techniques involving injection into mice of cells of a human squamous lung carcinoma cell line. PF/2A antibody is reported to react with breast carcinoma, colon carcinoma, gastric carcinoma, tumors of ectodermal origin and squamous lung cell carcinomas as well as a 24 kd polypeptide extracted from squamous lung cell carcinoma cells [Fernsten et al., Cancer Res., 46, 2970-2977 (June, 1986)]. Monoclonal antibody PF/2A is also reported to immunoprecipitate a 46 kd polypeptide extracted from human cell lines infected with Mycoplasma hyorhinis (M. hyorhinis), and stains, but does not precipitate, a 24 kd component derived from M. hyorhinis [Fernsten et al., Infect. Immun., 55, 1680-1685 (July, 1987)]. However, no association with regression of tumors is shown or suggested for this antibody or antigens reactive with the antibody by Fernsten et al.

## Summary of the Invention

Regression-associated antigens may be identified based upon their reactivity with regression-associated antibodies (RAAbs), which antibodies are produced in patients undergoing active immunization and responding with tumor regression.

A method for detecting RAAbs according to the present invention involves obtaining a first sample of serum from a patient diagnosed as not being in a state of regression and then obtaining a second sample of serum from the patient after diagnosis as being in a state of regression. Protein extracts of a neoplastic cell are exposed to each of the samples. The formation of an immune complex between a component of the neoplastic cell and an antibody in the second serum sample and the absence of such a complex with an antibody in the first serum sample is indicative of the presence of RAAbs in the second sample.

A method for purifying RAAs according to the present invention involves disrupting the neoplastic cell with associated RAAs (as determined by complex formation with an RAAb) into components of a solution, separating the component of the solution into fractions and identifying fractions containing RAAs by immune complex formation with RAAbs. A purified and isolated RAA according to the present invention has a molecular mass as determined by reducing SDS polyacrylamide gel electrophoresis to be within the range from about 19000 to about 23000 daltons, or within the range from about 38000 to about 45000 daltons or within the range from about 65000 to about 71000 daltons. Preferably these regression associated antigens having the above molecular masses do not bind to cationic exchange resins, such as DEAE Sephacel, at 50 mM $Na_3PO_4$ (pH 7) or to heparin agarose resins at 200 mM $Na_3PO_4$ (pH 7). More preferably, the regression associated antigen is greater than or equal to 95% pure as determined by reducing SDS PAGE analysis followed by silver staining.

The present invention also comprehends regression associated antigens encoded by DNA from a mammalian cell line which may have an infection with M. hyorhinis and regression associated antigens encoded by DNA of Mycoplasma hyorhinis.

The present invention provides the purification and a partial amino acid sequence analysis of two distinct molecular entities, a 38 kd and a 43 kd protein, from cultured M. hyorhinis and the demonstration that these two proteins are reactive with human regression-associated antibodies. The present invention involves the isolation and partial characterization of M. hyorhinis DNA sequences which encode all or part of the 38 kd antigen and the 43 kd antigen and the production of these proteins from cultured M. hyorhinis, mammalian cells infected with M. hyorhinis and anticipates the production of these antigens from genetically engineered microbial or mammalian cells containing M. hyorhinis gene sequences which encode the 38 kd and/or the 43 kd antigens.

Immunotherapy may be performed according to the present invention exposing to the immune system of a patient by introducing into a bodily fluid an RAA according to the present invention, a neoplastic cell containing one or more of the molecular species of RAAs according to the present invention, a cell-free preparation (e.g. particulate preparation or protein released in the culture fluid) from such a neoplastic cell, M. hyorhinis-derived protein preparations containing the 38 kd or the 43 kd antigen or protein preparations containing 38 kd and/or 43 kd antigen derived from genetically engineered cells containing a gene encoding the 38 kd or the 43 kd antigen or any portion thereof which includes an epitope. Such protein preparations may be exposed to the immune system of a patient by introducing them into the lymphatic or hematic fluid (i.e., the lymph or blood) or into tissues of a patient (e.g. by intramuscular or subcutaneous injection). The response of a patient to immunotherapy may be monitored for symptoms and signs associated with a neoplasm and by determining a circulating level of RAAbs in a patient.

The present invention also provides monoclonal or monospecific polyclonal antibodies exhibiting a specific immunoreactivity with an RAA. An antibody according to the present invention may be purified by contacting a substrate bound RAA with a solution containing an RAAb and eluting the RAAb from the RAA.

A monoclonal or monospecific polyclonal antibody directed against one or more of the RAAs of the present invention may be used in radioimmunoassays, enzyme-linked immunoadsorbent assays or direct or indirect immunohistochemical assays to determine the presence and the levels of one or more RAAs in tumor biopsy specimens or in body fluids. Such an antibody may also be administered to cause direct antibody-dependent tumor cell cytotoxicity (ADCC) or complement-dependent tumor cell cytotoxicity (CDCC). Alternatively, such an antibody may be bound to a bioactive moiety including but not limited to an anticancer drug, such as toxins, radioisotopes or chemotherapeutic substances, or cell growth and differentiation regulators (e.g., IL-1, TGF-$\beta$, TNF, IFN and the like) and introduced into a bodily fluid of a patient so that the active moiety is preferentially delivered to tumor cells by the specificity of the drug-bound antibody for the tumor cell. Such approaches may be useful in the in vivo diagnosis of or in the therapy of malignancies.

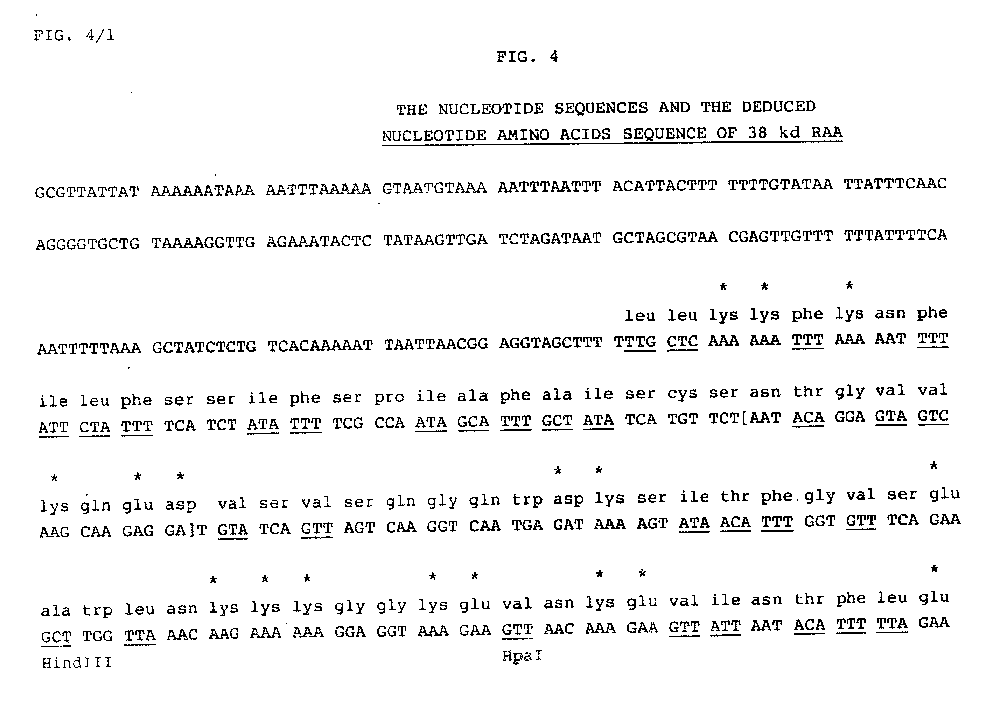

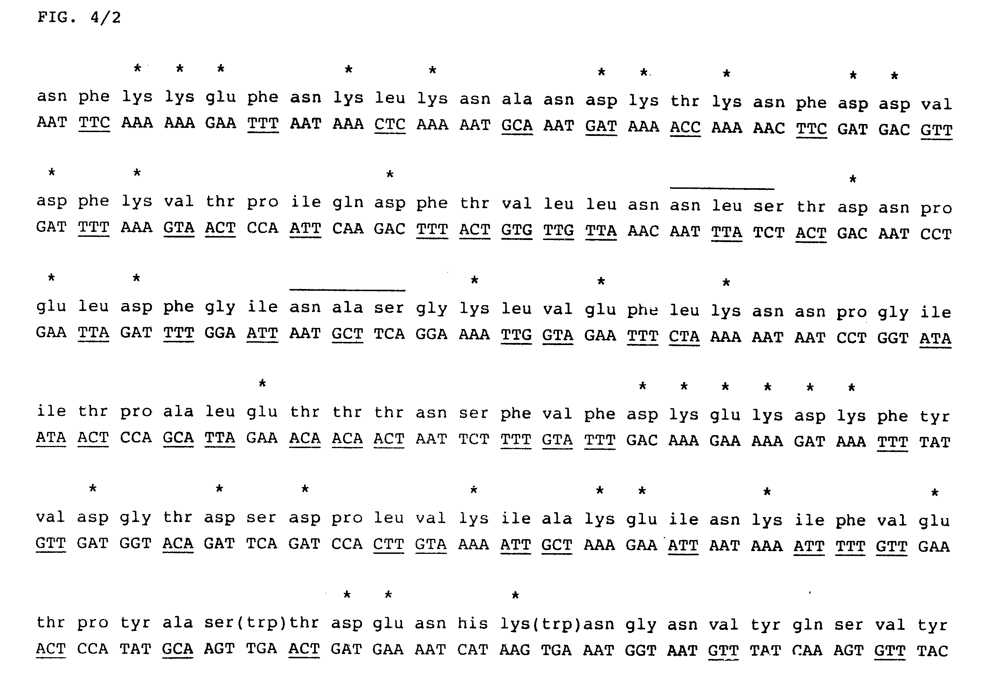

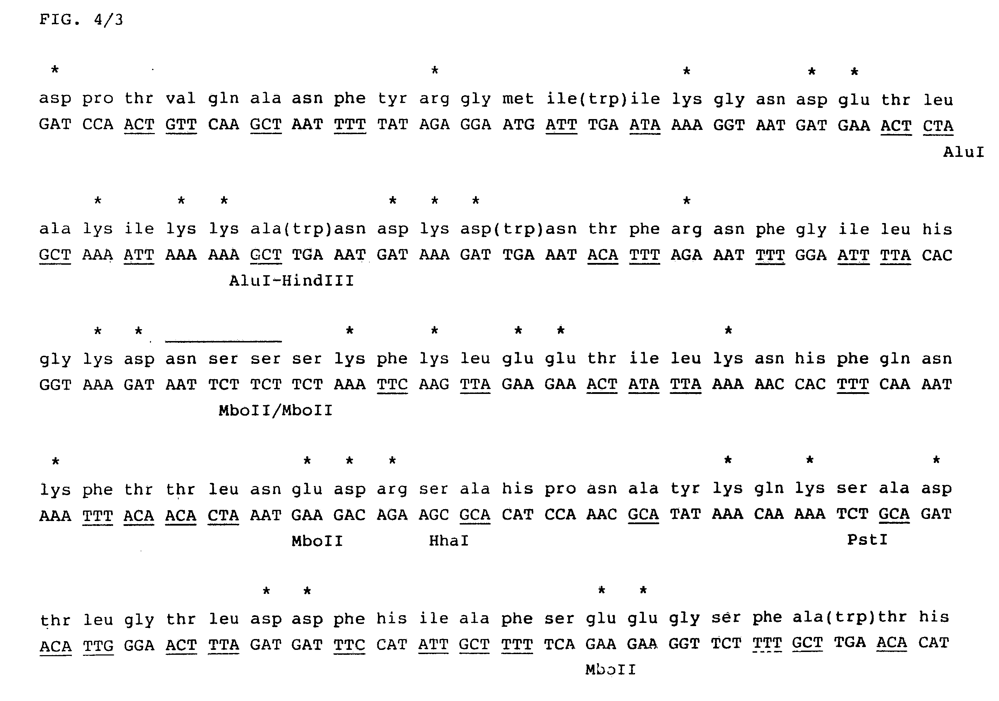

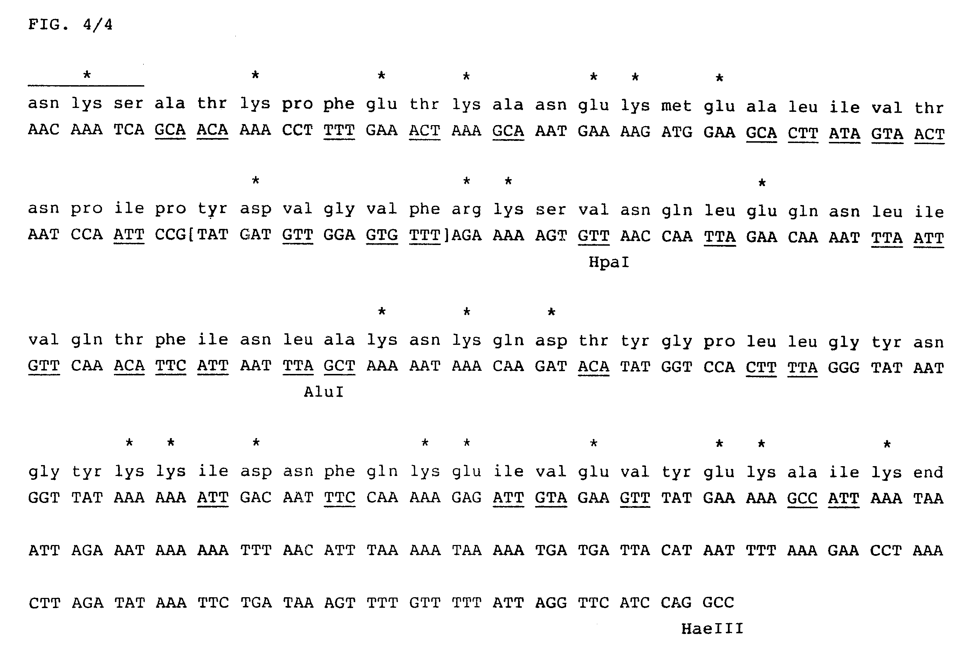

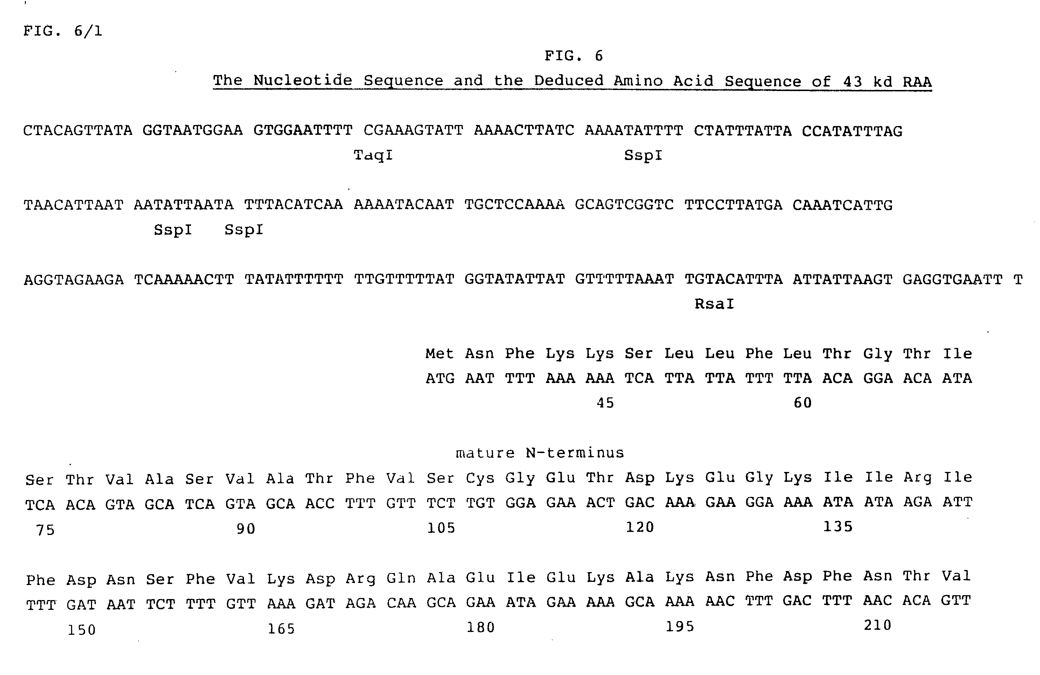

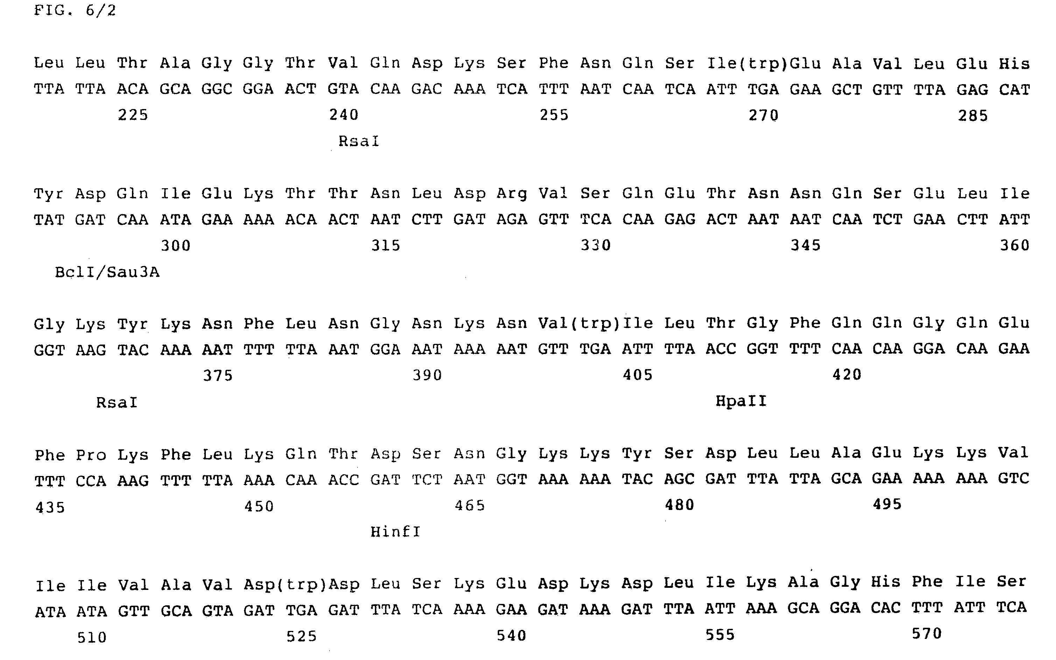

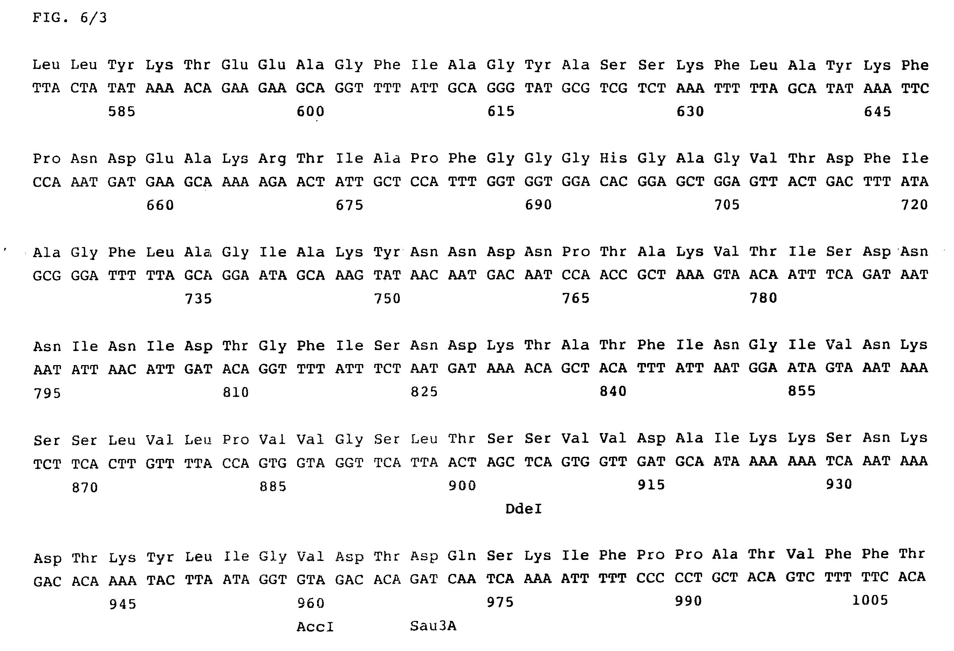

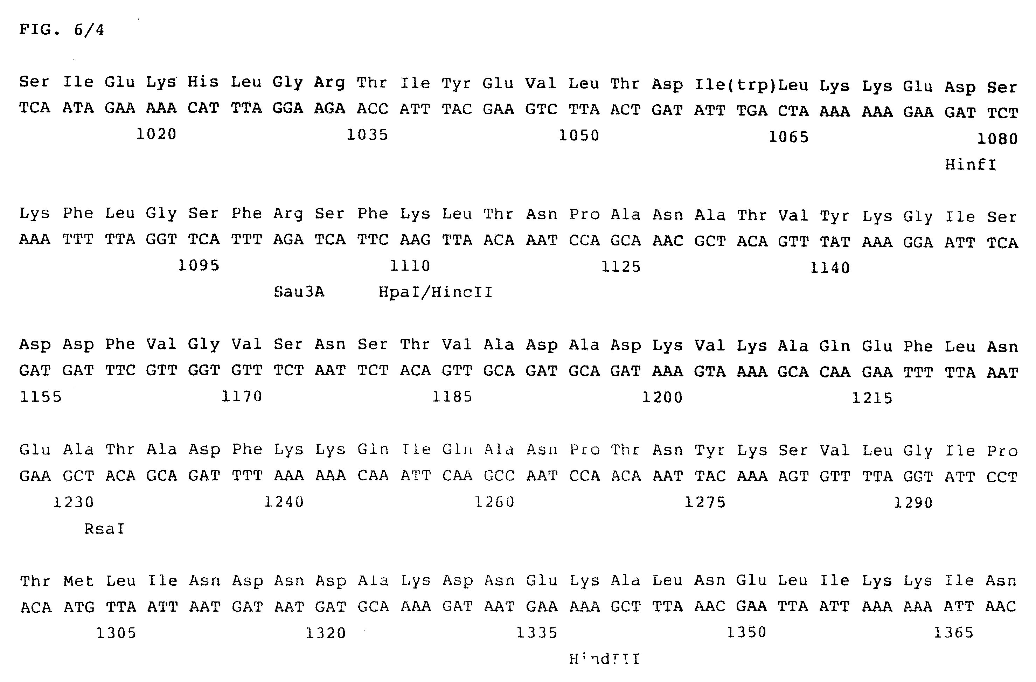

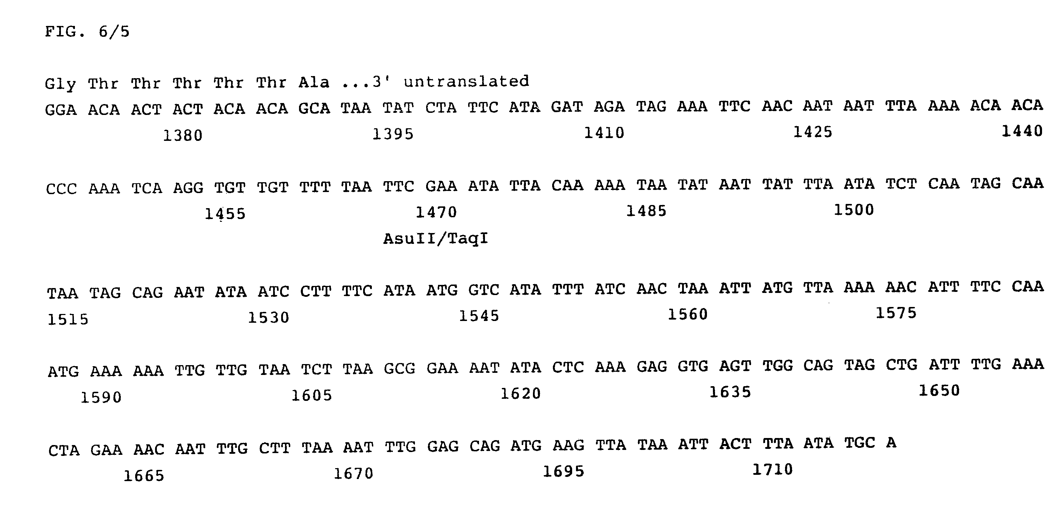

The present invention also provides a regression-associated antigen encoded by a purified and isolated nucleic acid described by a nucleotide sequence selected from the group consisting of: the nucleotide sequence as shown in FIG. 4 or 6; a nucleotide sequence which encodes the same sequence of amino acids as encoded by the nucleotide sequence shown in FIG. 4 or 6; a nucleotide sequence which hybridizes with any 20 sequential nucleotides as shown in the nucleotide sequence shown in FIG. 4 or 6; a nucleotide sequence which would hybridize with any 20 sequential nucleotides as shown in the nucleotide sequence shown in FIG. 4 or 6 but for the redundancy of the genetic code; and a nucleotide sequence which encodes an epitope encoded by 18 sequential nucleotides in the nucleotide sequence shown in FIG. 4 or 6. The present invention also provides a monoclonal or monospecific polyclonal antibody exhibiting a specific immunoreactivity with such an antigen.

A purified and isolated nucleic acid according to the present invention may be described by a nucleotide sequence selected from the group consisting of: the nucleotide sequence as shown in FIG. 4 or 6; a nucleotide sequence which encodes the same sequence of amino acids as encoded by the nucleotide sequence shown in FIG. 4 or 6; a nucleotide sequence which hybridizes with any 20 sequential nucleotides as shown in the nucleotide sequence shown in FIG. 4 or 6; a nucleotide sequence which would hybridize with any 20 sequential nucleotides as shown in the nucleotide sequence shown in FIG. 4 or 6 but for the redundancy of the genetic code; and a nucleotide sequence which encodes an epitope encoded by 18 sequential nucleotides in the nucleotide sequence shown in FIG. 4 or 6. The present invention also provides a cell transformed with such a nucleic acid, and an expression product of such a cell.

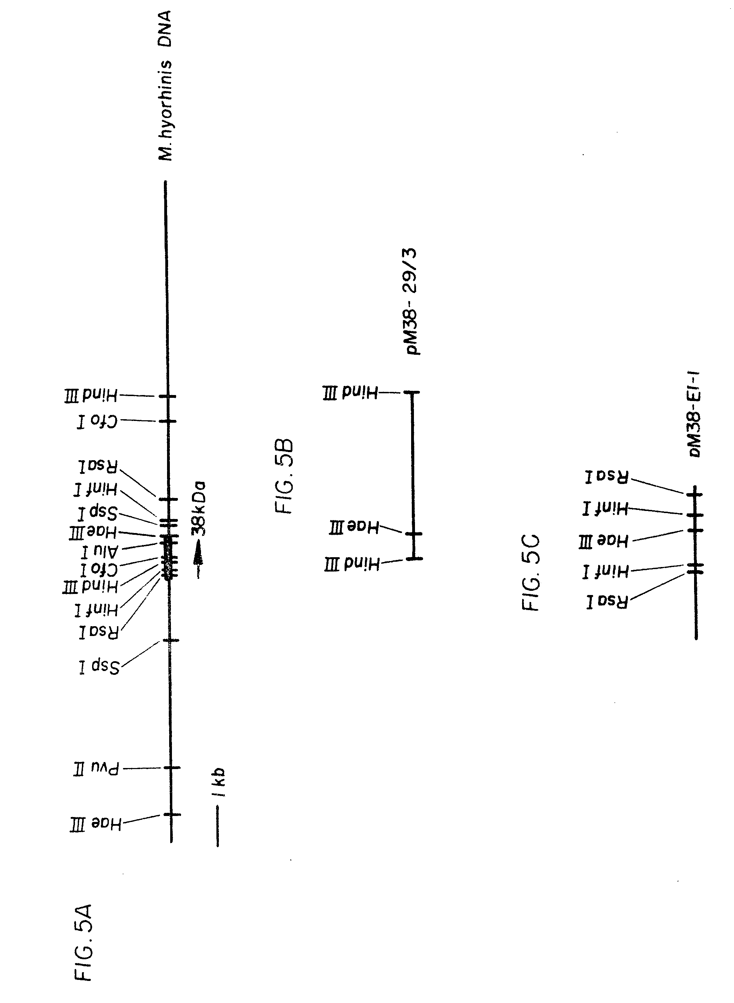

The present invention further provides a purified and isolated nucleic acid described by a restriction map shown in FIG. 5, a cell transformed with such a nucleic acid, and an expression product of such a cell, as well as a monoclonal or monospecific polyclonal antibody exhibiting a specific immunoreactivity with the expression product.

A method of in vivo imaging according to the prresent invention includes injecting an RAAb into a patient, the antibody being bound to a radioisotope, and scanning the patient using radioisotope scanning, or injecting an RAAb into a patient, the antibody being bound to a heavy metal, and scanning the patient using magnetic

4

resonance scanning.

A method for isolating a regression-associated antigen from M. hyorhinis according to the present invention includes extracting protein from cells of M. hyorhinis, separating from the the cells a regression-associated antigen encoded by a purified and isolated nucleic acid described by a nucleotide sequence selected from the group consisting of: the nucleotide sequence as shown in FIG. 4 or 6; a nucleotide sequence which encodes the same sequence of amino acids as encoded by the nucleotide sequence shown in FIG. 4 or 6; a nucleotide sequence which hybridizes with any 20 sequential nucleotides as shown in the nucleotide sequence shown in FIG. 4 or 6; a nucleotide sequence which would hybridize with any 20 sequential nucleotides as shown in the nucleotide sequence shown in FIG. 4 or 6 but for the redundancy of the genetic code; a nucleotide sequence which encodes an epitope encoded by 18 sequential nucleotides in the nucleotide sequence shown in FIG. 4 or 6; and a purified and isolated nucleic acid described by a restriction map shown in FIG. 5.

The present invention further provides an immortalized cell line producing a monoclonal antibody to a regression-associated antigen, in particular a cell line secreting an $I_gM$ antibody to a regression-associated antigen, and more particularly the cell line designated as ATCC Deposit No. HB 9540. A monoclonal antibody secreted by the cell line designated ATCC Deposit No. HB 9540 is also provided by the present invention.

An immunoassay kit according to the present invention includes a regression-associated antibody and may further include a regression associated antigen. Alternatively, an immunoassay kit according to the present invention may include a regression associated antigen in the absence of a polyclonal or monoclonal regression-associated antibody.

A method of passive immunization according to the present invention involves injection into a patient a regression-associated antibody.

The present invention also provides a regression-associated antigen encoded by a purified and isolated nucleic acid described by a nucleotide sequence selected from the group consisting of: a nucleotide sequence which encodes the sequence of amino acids Ser-Gly-Glu-Thr-Asp-Lys-Glu-Gly-Lys-Ile-Arg-Phe-Asp-Asn-X-Phe-Val-Lys-Asp wherein X may be Cys or Ser; a nucleotide sequence which would hybridize with any 20 sequential nucleotides encoding Ser-Gly-Glu-Thr-Asp-Lys-Glu-Gly-Lys-Ile-Arg-Phe-Asp-Asn-X-Phe-Val-Lys-Asp wherein X may be Cys or Ser but for the redundancy of the genetic code; and a nucleotide sequence which would hybridize with any 20 sequential nucleotides as shown in the nucleotide sequence encoding Ser-Gly-Glu-Thr-Asp-Lys-Glu-Gly-Lys-Ile-Arg-Phe-Asp-Asn-X-Phe-Val-Lys-Asp wherein X may be Cys or Ser; and a nucleotide sequence which encodes an epitope encoded by 18 sequential nucleotides in the nucleotide sequence encoding Ser-Gly-Glu-Thr-Asp-Lys-Glu-Gly-Lys-Ile-Arg-Phe-Asp-Asn-X-Phe-Val-Lys-Asp wherein X may be Cys or Ser.

<u>Brief Description of the Drawings</u>

FIG. 1 is a schematic depiction of immunoblots of serum dilutions for samples from two immunized patients (Pt. 1, Pt. 2) and an immunized rabbit tested against the particulate fraction (lanes numbered 1) and conditioned medium (lanes numbered 2) from A375 cells. Pt. 1 received intralymphatic (I.L.) immunotherapy with intact, irradiated tumor cells, Pt. 2 received subcutaneous (S.C.) immunization, and rabbit 209 was immunized subcutaneously with the particulate fraction of A375 (ING-A) cells;

FIG. 2 is a schematic depiction of an HPLC chromatogram of a partially-purified A375 (ING-A) extract and the corresponding immunoblot analysis of HPLC pools;

FIG. 3 is a schematic depiction of immunoblots in which regression associated antibodies from three different patients (A, B, and C) were allowed to react with antigens fractionated from cell lysates of early (MAC-1) and late (MAC-3) passages of two different melanoma cell lines;

FIG. 4 is a restriction map, nucleotide sequence and deduced amino acid sequence for a gene encoding the 38 kd RAA of M. hyorhinis and containing an 18 mer probe sequence and a 26 mer probe sequence according to the present invention;

FIG. 5A is a restriction map of a gene encoding a 38 kd protein of M. hyorhinis; FIG. 5B is a restriction map of a clone designated pM38-29/3 which contains sequences encoding the C-terminal region of the 38 kd RAA; and FIG. 5C is a restriction map of a clone designated pM38-El-l containing sequences encoding the N-terminal region of the 38 kd RAA and sequences 5' to the coding sequence; fragments in PM38-29/3 and PM38-El-l contain the homologous restriction sites found in the corresponding DNA segment indicated in FIG. 5A;

FIG. 6 is a restriction map, nucleotide sequence and deduced amino acid sequence for a gene encoding the 43 kd RAA of M. hyorhinis; and

FIG. 7A is a restriction map of a M. hyorhinis DNA fragment containing the gene encoding a 43 kd protein of M. hyorhinis; FIG. 7B is a restriction map of a clone designated pMu3-8 which contains sequences encoding the N-terminal region of the 43 kd RAA; and FIG. 7C is a restriction map of a clone designated pMu3-1 containing sequences encoding the 43 kd RAA and sequences 3' and 5' to the coding sequence. The DNA fragments in pm43-8 and pM43-1 contain the homologous restriction sites found in the corresponding DNA segment indicated in FIG. 7A.

Detailed Description

The present invention relates to the identification of human tumor cell-associated antigens and the properties of antibodies developed against such antigens in patients responding with tumor regression following active specific immunotherapy using tumor cells or cell extracts. These novel antigens, designated herein as RAAs, have been detected using RAAbs from patient sera in a number of fresh human tumor extracts and in cultured human tumor cell lines.

Discrete antigens associated with cultured human tumor cells may be identified by screening large numbers of monoclonal antibodies produced in mice and other animals against tumor cells or partially fractionated immunogens derived therefrom. However, most antibodies directed against such antigens may not be associated with the progression or regression of tumors and, as such, the antigens detected by these antibodies are unlikely to have therapeutic potential as active immunogens. Furthermore, human antigenic determinant(s) which elicit an antibody response in mice or other animals might not trigger human immune surveillance mechanisms against a tumor.

In the present invention, specific antibodies have been detected in sera from patients undergoing tumor regression following active immunization by intralymphatic infusion of cells derived from their own tumors (autochthonous) or established tumor-derived cell cultures of similar histopathologic type (allogeneic). The antibodies of this invention are characterized by their specific reactivity toward certain antigens associated with certain human tumor cells. These antibodies have thus been used to identify specific antigens in tumor cells and tissues. These tumor-associated antigens, designated herein as RAAs, may be further grouped based upon their sizes, their ability to react the RAAbs from patients regressing different malignancies, and their presence and/or their relative abundance in cells obtained from different types of human cancer.

RAAbs according to the present invention are antibodies which are induced in response to administration of irradiated tumor cell immunogens and are associated with the stabilization or regression of tumor masses.

RAAs according to the present invention include antigens present on human tumor cells which presumably induce the production of RAAbs and which thus may be recognized by antibodies in the sera of patients showing tumor regression in response to tumor cells administered through the intralymphatic route.

The present invention also concerns methods for obtaining preparations containing such RAAs including established human tumor cell lines, protein and cell-free tissue extracts and preparations derived from membranes or culture media of such cells and tissues. RAA's or proteins containing certain antigenic determinants present in RAA's may be encoded by the DNA of M. hyorhinis and may therefore, be isolated from cultured M. hyorhinis or cells expressing M. hyorhinis genes encoding such proteins. Polyclonal or monoclonal antibodies against RAAs may be prepared in animals and may be used for therapeutic purposes, diagnostic tests or monitoring the course of therapy, including therapy involving active immunization protocols.

Example 1

Specific Antibodies Against Tumor Cell-Associated Antigens In Patients Showing Evaluable Tumor Regression After Immunotherapy

1(a) Serum collection.

Serum samples are obtained from patients with documented malignancies that are in the state of tumor progression from the primary site of origin to other locations in the body (metastasis) or by a demonstrable growth of the primary tumor mass. The patients are then subjected to intralymphatic immunotherapy as described in Juillard et al., Bull. Cancer, 66, 217 (1979), which is incorporated by reference herein, using infusions of tumor cells obtained from their own tumors or cultured tumor-derived cells established from malignancies of the same type as the patient in question as described in Juillard et al., Cancer, 41, 2215 (1978); and in Weisenburger et al., J. Biol. Response Mod., 1, 57 (1982), both of which are also incorporated by reference herein. The amount of neoplastic cells used for immunization and methods of their processing including washing and irradiation prior to administration into patients is presented by Juillard and his colleagues in published reports including Juillard et al., Cancer, 41, 2215-2225, (1978) and is also described in Bubbers et al., Bull. Cancer, 68, 332-337 (1981).

Neoplastic cells used in immunization are often defined as cells derived from tumors isolated from cancer patients, which cells may have karyotype different from that of cells derived from corresponding normal tissues and may have chromosomal abnormalities and which cells, when allowed to proliferate in culture, may have altered metabolic and growth properties. These altered growth properties include one or more of the following: (a) the loss of anchorage-dependent growth and the ability to form masses in semi-solid media; (b) the ability to produce solid tumors in athymic mice; and (c) alterations in membrane structures, including the presence of tumor-associated antigens, examples of which are described in the literature and which are distinct from the antigens described in this invention. See Hood et al., Immunology, Benjamin/Cummings Publishing Co., 510-529, (1984); and Sell, in Proceedings of the UCLA Symposium on Monoclonal Antibodies and Cancer Therapy, supra, 3-21 (1985).

A state of tumor regression in patients undergoing intralymphatic immunotherapy described above is established by evaluating the status of the tumor using several criteria. The evaluation process comprises accepted clinical monitoring procedures including standard radiological evaluations, computerized axial tomography, magnetic resonance imaging, assessment of various immunological markers for cancers (e.g., carcinoembryonic antigen, newly discovered antigens detected by tumor-specific monoclonal antibodies), detection of tumor-specific enzymes and hormone receptors, and other standard laboratory tests used in the clinical management of cancer patients. In addition, a careful physical examination, including palpation of tumor nodules (wherever possible), is conducted.

Partial tumor regression is indicated if the following is observed: stabilization of a tumor which was progressing prior to immunotherapy (i.e., failure to detect any objective change in tumor size for three months), or less than fifty percent decrease in tumor size and associated subjective improvement or status quo. Such stabilization of tumor growth is associated with the development of delayed hypersensitivity to immunogens in the irradiated neoplastic cells used for treatment and is assessed by subcutaneous and intradermal skin testing of the cellular preparations.

A successful tumor regression response is defined as an objectively measurable decrease (i.e., at least fifty percent) in the size of the tumor mass. Tumor mass is assessed by direct measurement, when the tumor is near the surface of the body and directly palpable, by radiological measurements and by the additional criteria cited above.

Serum samples are obtained from patients before and at different times after initiation of the immunotherapy. The status of the tumor is assessed as described above. The serum samples from patients undergoing the immunotherapy regimen and responding with tumor regression are rested along with the serum samples from each patient obtained prior to the initiation of immunotherapy and/or before the patient is in a state of regression.

1(b) Human tumor cell lines.

The following human tumor cell lines were obtained from the American Type Culture Collection (ATCC), Rockville, Maryland, were propagated according to ATCC recommendations and were shown to express RAAs using the Western immunoblotting technique as described in section 1(d) below: A375 melanoma cells (ATCC No. CRL 1619); LoVo colonic adenocarcinoma cells (ATCC No. CCL229); A549 lung adenocarcinoma cells, (ATCC No. CLL185); and SW480 colonoic carcinoma cells (ATCC No. CCL228). Hereinafter, all references to A375 cells are to be understood to refer to the ING-A variant of A375 cells, cited above, which variant is deposited on February 12, 1987 as ATCC No. CRL 9321, with the American Type Culture Collection 12301 Parklawn Drive, Rockville, Maryland 20852. The method of detecting RAAs in tumor cells as described in this invention has also demonstrated the presence of RAAs in various other human tumor-derived cell lines including certain cell lines which were maintained by Dr. G. Juillard, University of California at Los Angeles are designated 69-2 or WRO 82-1, CAL-27 and CAL-33 (squamous cell carcinomas), 69-3 or CAL-2 and FR084-1 (ovarian adenocarcinomas), BR081-1 and RR081-1 (renal cell carcinoma) and SR084-1 and HR084-1 (prostatic carcinoma), 100P3, CR081-6, CR081-8 (lung carcinoma), MR081-1, MR084-1 (adenocystic carcinoma), M7, M6, M14, M20, M78, CR081-5, RR081-3, ER081-1, WR081-1 (melanoma), DR081-1, WR082-1, AR081-1 (thyroid carcinoma). These cell lines were established by conventional techniques using tissue specimens obtained from the respective tumors, and several subcultures have subsequently been carried out under standard human cell culture conditions leading to their growth for 50 or more passages without observable cytologic changes. Samples of each cell culture were evaluated by microbial culturing, cytology or electron microscopy for viral or microbial contamination, and were shown to be free of identifiable microorganisms with the exception of Mycoplasma hyorhinis (as determined by Coriell Institute, Camden, New Jersey).

Mycoplasma infection of mammalian cell lines is known to result in changes in cellular metabolism and function. Van Diggelen et al., Exp. Cell Res., 106, 191 (1977); and Van Diggelen et al., Cancer Res., 37, 2680 (1977). In addition, mycoplasmas tend to be strongly adherent to the surfaces of mammalian cells and would be present in soluble and particulate extracts from washed cells. Butler et al., Infect. Immun., 42, 1136 (1983). Because of the suspected presence of M. hyorhinis in the A375 melanoma cell line (ING-A) and all other RAA-positive cell lines described in Section 1(b), the involvement of this microorganism in the production of RAAs either encoded for in the M. hyorhinis genome or through induction of expression of RAAs in the host human tumor cells is a legitimate possibility for experimental determination.

Two cell lines were established at INGENE from a 64 year old white female who presented with metastatic ovarian adenocarcinoma and involvement of inguinal lymph nodes. The first cell line, ING 69-1, est established from a one gram surgical biopsy specimen of an inguinal lymph node. The specimen was minced using sterile scissors, passed through a gauze mesh and incubated for one hour at 37°C with collagenase followed by an

7

incubation with trypsin for an additional 30 minutes at 37°C. The tumor cell suspension was rinsed in cell culture medium, plated in RPMI 1640 medium (as above) and subcultured to generate sufficient numbers of cells for the active immunization of this patient [Weisenburger et al., J. Biol. Response Mod., 1, 57-66 (1982)]. Approximately 9 months after ING 69-1 was established, this patient underwent surgical removal of abdominal metastatic ovarian tumor masses and ING 69-11 cell cultures were established from tumor cells in ascites fluid from this patient.

1(c) Preparation of total cell or tissue extracts.

Normal or neoplastic cells obtained from confluent cell cultures or freshly minced tissue biopsies were rinsed twice with phosphate buffered saline and then extracted using a mixture of ionic and non-ionic detergents according to the procedure of Sen et al., Proc. Nat'l Acad. Sci. (USA), 80, 1246-1250 (1983), or solely using a non-ionic detergent buffer which preferentially extracts proteins from cell membranes [10 mM Tris-HCl, pH 7.5, 1 mM EDTA, 2.5 mM EGTA, 10 mM NaMoO$_4$, 12 mM monothioglycerol, 10% glycerol, 40 µg/ml leupeptin (Sigma Chemical Company, St. Louis, Missouri) and 0.2% Triton X-100®]. Insoluble materials were removed by centrifugation at 10,000 X g and the supernatant solutions were retained. Aliquots of the supernatants containing 10 to 20 µg of cell protein were subjected to reducing sodium dodecyl sulfate-polyacrylamide gel electrophoresis (SDS-PAGE) essentially according to the procedure as described in Laemmli, Nature, 227, 680-685 (1970).

1(d) Immunoblotting.

The detection of discrete RAAs is achieved using a Western immunoblotting technique where antibodies, RAAbs, from a patient serve as probes to detect specific antigens. Screening proteins obtained from a variety of normal and tumor cells with antibodies from a number of patients exhibiting tumor regression allows the identification of molecular characteristics of antigens which are detected by RAAbs in patients regressing tumors. It follows that these antigens elicit the production of RAAbs, the antibodies associated with tumor regression.

The immunoblotting procedure consists of the following steps as described in Towbin, et al., Proc. Nat'l Acad. Sci (USA), 76, 4350 (1979), which reference is incorporated by reference herein. In a typical Western immunoblotting procedure, total cell protein extracts or subcellular fractions are subjected to reducing sodium dodecyl sulfate-polyacrylamide gel electrophoresis (SDS-PAGE). The proteins, which may be RAAs, are transferred by electroelution onto nitrocellulose filters which are subsequently incubated with appropriate dilutions of the test serum or antibody preparation. After the incubation of the filter with antibody, which contain RAAbs, and extensive washing, the filter is incubated with I[125]-labeled Protein A of Staphylococcus aureus (which specifically binds to the Fc region of antibody molecules), washed to remove unbound Protein A and exposed to X-ray film for autoradiography. Each radioactive band represents the location of a protein species which formed an immune complex with antibodies from the test sera.

1(e) Patients, treatments and antibody responses: Evaluation of independent serum samples from certain patients.

Immunoblots were performed using pre-immune sera and serum samples (post-immune sera) obtained at various times after the initiation of immunotherapy and at a time when tumor regression was noticeable and post-immune sera from three different patients including the previously described patients. All patients underwent intralymphatic immunotherapy using cell lines established from their own tumors and other cultured cells established from related tumors from other patients. Antigens having molecular weight of approximately 70,000 daltons and 43,000 daltons were readily detected in protein preparations from malignant cells of ovarian origin using the post-immune sera from these three patients while no significant immunoreactivity was observed with the pre-immune sera from the same patients. The post-immune sera of these patients did not react with antigenic species in various normal human tissue or cultured cell extracts including those from normal breast and ovary.

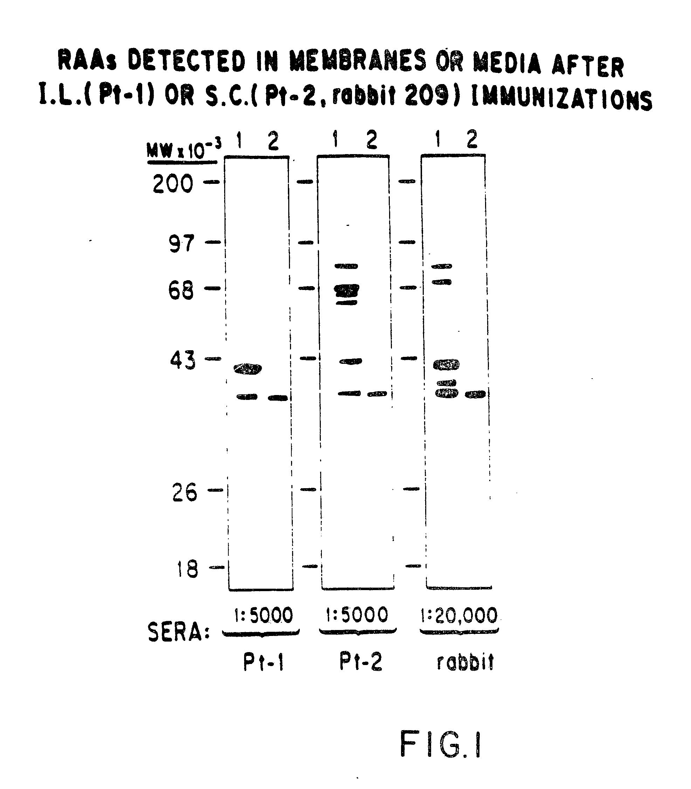

Results of immunoblot studies using post-immune sera and ascites fluid from patient A (ovarian cancer) indicated the presence of comparable antibodies in both as detected by their reactivities with a 70 kd antigen band and with the antigen bands of 38 kd to 40 kd and 43 kd to 45 kd present in several different ovarian and non-ovarian tumor cell lines. FIG. 1 presents immunoblots using 1:1000 sera dilutions from 2 immunized patients (Pt-1 and Pt-2) and a rabbit (#209) immunized with the particulate fraction obtained from A375 (ING-A) cells; all sera were tested against the particulate fraction (lanes labeled 1) or conditioned medium (lanes labeled 2) from A375-ING-A cells. The patterns of immune complex formation in this test represent what is typically observed with sera from patients regressing tumors. It was also evident from the immunoblot data that the antigen species preliminarily identified to be of about 43 kd included at least two entities with molecular weights of 38 kd to 40 kd (the "38 kd" antigen) and 43 kd to 45 kd (the "43 kd" antigen) being resolved by SDS-PAGE system of higher resolving power. Both of these antigenic species appear to be significantly different from the major histocompatibility antigens (HLA-ABC) in that their mobilities in reducing SDS-PAGE were unaffected by treatment with endoglycosidases H and F under conditions where the carbohydrate moieties of HLA-ABC is degraded by these enzymes (as visualized by immunoblots of these antigens using monoclonal antibodies specific for HLA-ABC to locate antigen bands). The 70 kd, 43 kd and 38 kd antigens are all also distinguishable from HLA-ABC in two dimensional gel electrophoresis.

A patient with metastatic prostatic cancer who received intralymphatic infusions of two irradiated cultured cells established from two independent prostatic cancers, SR084-1 and HR084-1, developed antibodies reactive with the 38 kd and the 43 kd antigens in the primary cell suspension of prostate tumors from which the HR084-1 cell line was derived; these antigens also were detected in SR084-1 cultured cells. Associated with this patient's immunotherapy were dramatic declines in serum acid phosphatase levels measured during the first two weeks following initiation of the immunizations.

The 43 kd and the 70 kd antigen species also were detected in certain fresh tumor extracts by the post-immune sera from several patients. The test extracts used in these studies were obtained from a non-Hodgkin's lymphoma and a breast carcinoma. Sera samples from a metastatic renal cancer patient were obtained before and after intralymphatic immunizations with a mixture of allogeneic renal cancer cell lines. Pre-immune serum from this patient showed negligible reactivity in the 43 kd antigen reaction with either of the two extracts tested; in contrast, the post-immune sera from this patient reacted with a specific 43 kd band in the lymphoma extract. In addition, a dog immunized with a human squamous cell carcinoma culture (69-2), also developed antibodies which reacted with specific antigens in the 68 kd to 70 kd range from a human Kaposi's sarcoma extract. The pre-immune sera from the patient and the dog were not reactive with the Kaposi's sarcoma extract.

Using techniques essentially as described above, antibodies in sera samples obtained from a large number of tumor-regressing patients have detected one or more of the three major molecular groups of RAAs with apparent molecular weights of about 68,000 daltons, about 43,000 daltons (also see below) and about 20,000 daltons in various tumor cells including tumor cell preparations used for immunization. Antibodies in sera collected prior to the initiation of immunotherapy failed to detect any of these three RAAs.

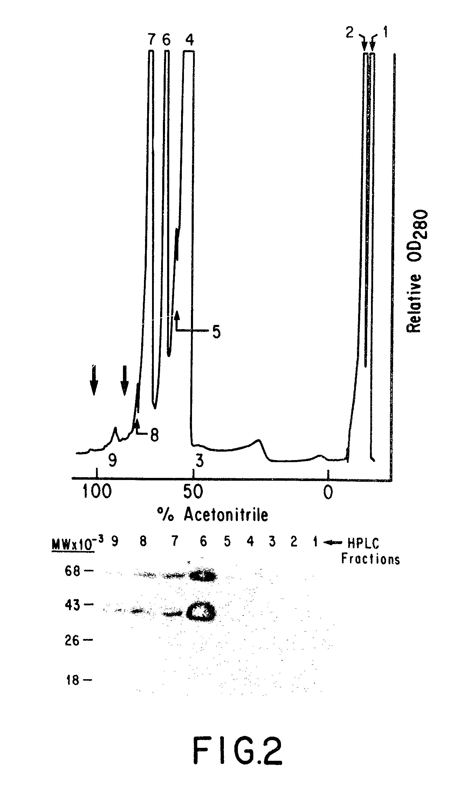

In order to further characterize these novel antigens, cell particulate fractions from human tumor cells have been used as the starting material to partially purify them. After extraction of the ING-A variant of A375 melanoma cells, ammonium sulfate fractionation and enrichment using affinity chromatography with RAAbs bound to sepharose, the 38 kd, 43 kd and 70 kd antigens were found to co-elute in an acetonitrile gradient using high performance liquid chromatography (HPLC). FIG. 2 illustrates the elution of these antigenic species as a major peak from a C-8 reverse phase HPLC column (this peak is labeled number 6 and an immunoblot analysis of antigen species in pools 1 to 9 is shown below the column profile wherein corresponding lanes are numbered to indicate correspondence to the numbered HPLC pools).

The approximate sizes of the RAAs have been estimated solely based upon the mobility, in reducing SDS-PAGE, of each of the detected RAAs relative to commercially available, pre-stained molecular weight size markers, Midrange Kit, purchased from Bethesda Research Laboratories, Inc., Bethesda, Maryland, or Diversified Biotech, Newton Centre, Massachusetts. One RAA with an assigned molecular weight of 68,000 daltons (68 kd) migrates within a size range of between 65 to 71 thousand daltons. Another RAA consists of either a single protein band of approximately 43,000 daltons (43 kd) in size within a range of between 41 to 45 thousand daltons, or frequently consists of a doublet having two detectable bands visualized on an immunoblot. The third species is an antigen of approximately 20,000 dalton molecular weight (20 kd) within a range of between 19 to 23 thousand daltons.

RAAs usually have been detected in 10 to 20 μg total protein preparations obtained from human tumor tissue and cell extracts using between 200- and 2000-fold dilutions of sera from patients regressing a variety of human malignancies including ovarian carcinoma, thyroid cancer, malignant melanoma, prostate cancer, epidermoid cancer, squamous cell carcinoma and certain others.

Table 1 summarizes results obtained with sera from five patients showing evaluable regression of the indicated metastatic cancers following intralymphatic immunotherapy. A serum sample obtained from each of the five patients (during regression) was used to perform immunoblotting studies on one or more tumor tissue extracts (designated by letter symbols) or extracts of established and primary cell cultures derived from various human tumors. The samples tested by each patient's serum sample are listed under the respective patient number. Some of the primary cell suspensions or established cultured cells have been used for intralymphatic immunotherapy and thus their abilities to induce tumor regression have been tested. These results are summarized in the second column as ILI status identified as follows: Rg+, indicating that the cell lines elicited tumor regression in ILI patients, Rg- indicating that the cell lines failed to elicit tumor regression in ILI patients, or NT indicating that the cell lines were not tested. For each of the three RAA species discussed in this table, a " + " indicates an easily detected signal in a standard immunoblot assay with the serum antibodies reacting with antigens present in 20 micrograms or less of total cellular proteins; a dilution of 200-fold or more of the sera from the indicated patient was used in all these studies. In Table 1, patient #1 had malignant melanoma, #2 had ovarian carcinoma, #3 had squamous cell carcinoma of the esophagus, #4 had prostate carcinoma and #5 had thyroid carcinoma.

## Table 1

### DISTRIBUTION OF RAAs IN TUMOR CELL LINES

| Cell line or tumor extract | Ability to Induce Regression | 43 kd RAA | 68 kd RAA | 20 kd RAA |
|---|---|---|---|---|
| PATIENT#1 | | | | |
| extract F | NT | + | + | − |
| M14 | Rg+ | + | − | − |
| 69-12 | Rg− | − | − | − |
| A375 | Rg+ | + | + | + |
| | | | | |
| PATIENT #2 | | | | |
| extract 0 | NT | − | + | − |
| 69-1 | Rg+ | + | + | − |
| 69-3 | Rg+ | + | + | − |
| 69-11 | Rg− | − | + | − |
| FRO81-2 | Rg+ | + | − | − |
| A375 | Rg+ | + | + | + |
| | | | | |
| PATIENT#3 | | | | |
| extract L | NT | − | + | − |
| 69-2 | Rg+ | + | + | − |
| 69-13 | Rg+ | − | + | − |
| SRO82-3 | NT | − | + | − |
| | | | | |
| PATIENT#4 | | | | |
| extract P | NT | + | + | − |
| SRO84-1 | Rg+ | + | + | − |
| | | | | |
| PATIENT #5 (cancer) | | | | |
| ARO81-1 | Rg+ | + | − | − |
| DRO82-1 | Rg+ | + | − | − |

10

In all of the patients presented in Table 1, immunoblotting analysis carried out with pre-immunotherapy sera failed to detect the presence of antibodies reacting with any of the respective RAAs. In several other cases evaluated, with the exception of two patients with malignant melanomas, the results of immunoblotting analysis carried out with pre-immunotherapy serum failed to reveal radioactive bands at about 68 kd, at about 43 kd or at about 20 kd, suggesting that most cancer patients prior to therapy do not contain antibodies against the RAAs of this invention. The detection of antibodies against the 38 kd and the 43 kd RAAs in the two melanoma patients prior to treatment with intralymphatic immunotherapy may be indicative of the known ability of melanomas to occasionally undergo spontaneous regression and to mediate such regression through the patients ability to generate such antibodies without active immunization.

The molecular mass determinations for the RAAs identified in Table 1 were made using "mini" SDS-PAGE in which 10% SDS-polyacrylamide resolving gels having a height of 7.5 cm are employed. When the "mini" SDS-PAGE was calibrated with molecular mass standards, the estimated principle sizes of the RAAs were of 68,000 daltons, 43,000 daltons and 20,000 daltons, although the doublet near 43,000 daltons was often not well-resolved in these gels. More precise molecular mass estimates have been obtained using the higher resolving power of a longer [14 cm] 10% SDS-polyacrylamide gel (as are employed in Fig. 1). Such gels calibrated with midrange molecular weight markers of molecular wights between 12 kd and 95 kd have indicated the molecular mass estimate to be 70,000 daltons rather than 68,000 daltons for the larger RAA and also has clearly resolved the 43,000 dalton doublet into two species: 43,000 daltons (43 kd) and 38,000 daltons (38 kd). Through the use of the higher resolving SDS-PAGE, the 43 kd RAA identified in Table 1 for patients No. 1, 2 and 4 was shown to contain large amounts of the 38 kd RAA.

In additional studies of patients subjected to immunotherapy but in whom no clinical responses were observed (i.e., in whom progression of the malignant condition continued as judged by clinical criteria including radiological analysis or direct physical examination), the results were analogous to those obtained using sera from patients prior to immunotherapy. Sera from many non-responding patients with several different types of malignancies were evaluated at different times after initiating intralymphatic immunotherapy and all of these failed to react with any of the aforementioned RAAs in extracts of tumor tissues and tumor cell lines tested.

The simple method of immunoblotting using serum samples from patients on extracts of tumor cells which contain RAAs as demonstrated by experimental results of this invention allows one to monitor the success of an immunotherapy protocol with regard to specific immune response to tumor cell-associated antigens. The monitoring may be accomplished by examining RAAb titer(s) (defined as the maximal serum dilution able to detect RAAs in a standard immunoblot procedure as described below) and RAAb specificities (defined as the apparent molecular masses of the specific antigens recognized by the serum samples).

Antibodies against tumor cell-associated components developed in patients immunized by the intralymphatic route with irradiated human tumor cell lines. These antibodies may be useful in the characterization of appropriate tumor cells or subcellular components for active specific immunotherapy of various cancers and were identified as follows.

## 1(f) Screening of antibody response in a large number of patients.

More than fifty cancer patients were treated with a mixture of autologous human tumor cells and allogeneic human tumor cell lines by intralymphatic administration according to procedures described above. In Table 2, is a detailed presentation of patients with regard to the type of cancer, the cell lines administered, the antigens detected by their sera after immunotherapy and their clinical outcome. Cellular antigens observed in a substantial number of patients who underwent immunotherapy included membrane antigens of molecular weight 70 kd, 43 kd and 38 kd using the higher resolving SDS-PAGE for molecular weight assignment. A few other antigens with various sites of cellular localization have been noted in isolated cases and have been listed in Table 2.

In Table 2, + = remission, 0 = stable, - = progression and ? = unknown. Delayed hypersenitivity skin tests were evaluated 24 and 48 hours after intradermal injection of $10^5$ irradiated tumor cells: S = small (5-10 mm diameter of induration and erythema), M = medium (10-25 mm diameter of induration and erythema) and N = negative. Tumor designations are the following: ov = ovarian, Sq = squamous cell, ENT = head and neck, pan = pancreas, AdLu = adeno carcinoma of lung, mel = melanoma, thy = thyroid. Antigens are indicated by the number corresponding to their size in kilodaltons. Where the letter "c" follows the antigen number the antigen identified was cytoplasmic in localization (e.g., 70c). Where the letter "n" follows, the antigen number, the antigen identified was nuclear in localization (e.g., 43 n). Where the number alone is listed (e.g. 43), the antigen is localized in the tumor cell membrane. "Cell lines" are human tumor cell lines used for immunization. The cell lines are designated with letter and number codes and cell suspensions prepared from fresh antologous tumor biopsies are designated RTM alone or RTH followed by a number indicates the clinical response could not be evaluated. A small number of patients show reactivity with a 68 kd antigen in addition to reacting with the 70 kd species. This 68 kd species has not yet been directly associated with tumor regression.

## Table 2

### Summary of Clinical and Immune Response
### Of Patients as Grouped by a Cell Line Which
### Was Common Among Those Used for Immunization

69-2

| Patient | Tumor | Cell Lines | Antigens | Response | Skin Test |
|---|---|---|---|---|---|
| 32 | Ov | 69-2 69-11T3 MCF-5 69-1 | 38, 43, 70 | 0 | |
| 21 | Sq | 69-2 CAL-33 | 38, 43, 70 | 0 | |
| 23 | Sq | 69-2 A375 | 38, 43, 70 | 0 | |
| 56 | Sq | 69-2 100P3 Cal27 RTH | 70c | − | S |
| 28 | Sq | 69-2 | | − | S |
| 14 | ENT | 69-2 CAL-27 100P3 | ? | ? | |
| 15 | ENT | 69-2 CAL-27 100P3 | 68, 70 | ? | |
| 16 | ENT | 69-2 CAL-27 100P3 | 43 | ? | |
| 17 | LUNG | 69-2 CRO81-6 100P3 CRO-81-B | | − | |
| 18 | Gast | 69-2 A375 RTH1719 | | − | M |
| 20 | Brea | 69-2 A375 RTH964 | 38, 43, 70 | + | |
| 40 | Lung | 69-2 RTH 1717 A375 | | ? | |

EP 0 308 265 A2

69-2 (cont'd.)

| Patient | Tumor | Cell Lines | Antigens | Response | Skin Test |
|---------|-------|------------|----------|----------|-----------|
| 46 | Lung | 69-2 A375 | | ? | |
| 19 | Pan | 69-2 A375 RRO81-4 | | – | S |
| 57 | AdLu | 69-2 M-6 CRO81-6 | | 0 | N |

A375

| Patient | Tumor | Cell Lines | Antigens | Response | Skin Test |
|---|---|---|---|---|---|
| 22 | Sq | A375 MRO81-1 MRO84-1 | 38, 43, 70, 43c | 0 | M |
| 23 | Sq | A375 69-2 | 38, 43, 70 | 0 | |
| 1 | Mel | A375 CRO81-5 | 38, 43 | + | S |
| 2 | Mel | A375 CRO81-5 M14 WRO81-1 | 18, 41, 43, 70 | + | S |
| 29 | Thy | A375 DRO81-1 WRO82-1 RTH1722 | 38, 43, 70 | – | M |
| 10 | Mel | A375 CRO81-5 Rth | 38, 43, 70 | 0 | S |
| 18 | Gast | A375 69-2 RTH1719 | | – | M |
| 20 | Brea | A375 69-2 RTH964 | 38, 43, 70 | + | |
| 40 | Lung | A375 RTH 1717 69-2 | | ? | |
| 46 | Lung | A375 WO 69-2 | | ? | |
| 19 | Pan | A375 69-2 RRO81-4 | | – | S |
| 58 | Mel | A375 CRO81-5 RTH | | 0 | S |

EP 0 308 265 A2

$\underline{100P_3}$

| Patient | Tumor | Cell Lines | Antigens | Response | Skin Test |
|---|---|---|---|---|---|
| 24 | Sq | 100P3 | 43 | + | S |
| 56 | Squ | 100P3 69-2 Cal27 RTH | 68c | − | S |
| 14 | ENT | 69-2 CAL-27 100P3 | ? | ? | |
| 15 | ENT | 69-2 CAL-27 100P3 | 68, 70 | ? | |
| 16 | ENT | 69-2 CAL 27 100P3 | 43 | ? | |
| 17 | Lung | 69-2 CRO81-6 100P3 CRO81-8 | | − | |

EP 0 308 265 A2

M-14

| Patient | Tumor | Cell Lines | Antigens | Response | Skin Test |
|---|---|---|---|---|---|
| 2 | Mel | A375 CRO81-5 M14 WRO81-1 | 18, 38, 43, 70 | + | S |
| 3 | Mel | M14 M7 M20 CRO81-5 RRO81-3 | 43c | + | N |
| 8 | Mel | M-78 M7 M14 M20 | | 0 | N |
| 47 | Mel | M14 CRO81-5 RTH-1513 | 43, 43c | − | N |
| 27 | Mel | ERO81-1 M14 | | 0 | |
| 6 | Mel | M7 M14 M20 | | − | S |

EP 0 308 265 A2

EP 0 308 265 A2

CAL33

| Patient | Tumor | Cell Lines | Antigens | Response | Skin Test |
|---------|-------|------------|----------|----------|-----------|
| 21 | Sq | 69-2 CAL 33 | 38, 43, 68 | 0 | |

CRO81-5

| Patient | Tumor | Cell Lines | Antigens | Response | Skin Test |
|---|---|---|---|---|---|
| 1 | Mel | A375 CRO81-5 | 38, 43 | + | S |
| 2 | Mel | A375 CRO81-5 M14 WRO81-1 | 38, 43, 68, 18 | + | S |
| 3 | Mel | M14 M7 M20 CRO81-5 RRO81-3 | 43c | + | N |
| 4 | Mel | CRO81-5 M14 | | 0 | |
| 7 | Mel | M-7 M-14 CRO81-5 | | + | |
| 9 | Mel | M-7 M-14 CRO81-5 WRO82-1 | | ? | |
| 10 | Mel | A375 CRO81-5 RTH | 38, 43, 68 | 0 | S |
| 42 | Mel | CRO81-5 M-14 RTH 1746 | | 0 | S |
| 58 | Mel | A375 CRO81-5 RTH | | 0 | S |
| 47 | Mel | M14 CRO81-5 RTH 1513 | 43, 43c | – | N |

EP 0 308 265 A2

As shown in Table 2, the majority of the patients who had progression of their tumors failed to elicit an antibody response to the 38 kd, 43 kd, and 70 kd antigens present primarily in extracts of A375 melanoma cells and 69-2 cells (squamous cell carcinoma of the esophagous).

In a typical immune response after intralymphatic immunotherapy (ILI), tumor cell-associated antigens having molecular weights of approximately 70 kd and 43 kd were readily detected in malignant cells of ovarian origin using the post-immune sera from patients numbered 23 and 52 in Table 2. No significant immunoreactivity was observed with the preimmune sera from the same patients. These antigens were present in the particulate fraction of A375 cell lysates as assessed by Western immunoblots.

The majority of the patients showing evaluable response developed antibodies to particulate-associated antigens having molecular masses of 38 kd, 43 kd and 70 kd. In several cases, however, the positive clinical response could not be correlated with the appearance of these antibodies.

As shown in Table 2, eight of twelve patients treated with A375 cells along with other cells responded to the therapy. The tumors progressed in two patients, and in two other, the clinical response could not be ascertained. Of the eight positive responders, seven developed antibodies to the particulate antigens having molecular weights of about 38 kd, 43 kd, and 70 kd. None of the non-responders or the ones of questionable status developed any antibodies to the membrane antigens.

Fifteen patients were treated with 69-2 cells along with other cells. Of these, only five showed a positive clinical response, five did not respond to the therapy, and in the remaining five patients the clinical status could not be ascertained. Four of the five positive responders had developed antibodies to membrane antigens 38 kd, 43 kd, and 70 kd. The majority of the other patients did not develop antibodies to the membrane antigens.

### 1(g) Antibody response and clinical outcome.

Determination of specificities and titers of RAAbs from over 50 patients undergoing intralymphatic immunotherapy have served to monitor the correlation between the development of antibodies to one or more of the RAA's of this invention and tumor regression or stabilization.

Tables 3 and 4 summarize the data which indicate an association of elevated RAAbs (anti-regression antibodies) with clinically evaluable regression or stabilization in the patients described in Table 2.

## Table 3

RAAbs and Clinical Response in Patients
Undergoing ILI with Allogeneic and/or
Autochthonous Tumor Cells.

| RAAb Antibody Response | Number of Patients | Clinical Response | | |
|---|---|---|---|---|
| | | Regression | Stabilization | Progression |
| Strong and Broad | 10 | 4 | 6 | 0 |
| Marginal and Narrow | 15 | 3 | 3 | 9 |
| Negative | 25 | 3 | 9 | 13 |

## Table 4

RAAbs and Clinical Response in Patients
Undergoing ILI with One or More of the Cell
Cultures (A375-ING-A and/or 69-2 cells)
Chosen For RAA Isolation.

| Antibody Response | Number of Patients | Clinical Response | | | |
|---|---|---|---|---|---|
| | | Regression | Stabilization | Progression | Unknown |
| Anti-RAA | 13 | 5 | 5 | 2 | 1 |
| No Anti-RAA | 17 | 1 | 6 | 6 | 4 |

EP 0 308 265 A2

**1(h) Identification of RAAbs in melanoma patients immunized subcutaneously with melanoma cell lysates.**

Two cell cultures derived from melanoma tissues of two individual female patients were combined after a final passage for 4 days in serum-free medium. A mechanical lysate was prepared with a Potter-Elvejhem homogenizer at 4°C and the lysate was diluted to a known concentration of tumor cell equivalents (i.e., an amount of lysate corresponding to the number of intact cells present prior to cell lysis, generally $10^7$ to $10^8$ cell equivalents per milliliter).

A first batch of lysate vaccine was called MAC-1 and used to treat an initial 8 patients with widespread melanoma. Based upon results of certain tests, MAC-1 cells may have been contaminated with Mycoplasma hyorhinis. Another batch, prepared in a very similar way with later passages of the cultured melanoma cells was called MAC-3 and used to treat the remaining patients in the study. MAC-3 cells do not appear to have been contaminated with Mycoplasma hyorhinis. The melanoma cell lysates were administered subcutaneously to patients along with an experimental adjuvant called DETOX® ( a detoxified fraction mycobacteria consisting principally of monophosphoryl lipid A, supplied by Ribi Immunochem Research, Inc., Hamilton, Montana). The adjuvant is known to have immune-stimulating activity and, therefore, the clinical response observed in melanoma patients may be due, at least in part, to an adjuvant effect (non-specific) in addition to the active immunization process.

Immunoblot studies were conducted using the post-immune sera from patients treated with MAC-1 (patients #1 to 8 the results are presented in Table 5) and MAC-3 (patients #9 to 15) to screen three different antigen preparation. The molecular masses in kilodaltons of the antigens detected by each patient sera are indicated under each extract. Clinical responses are indicated by : + = extensive regression, 0 = stabilization or partial regression, - = progression, and ? = not able to be evaluated.

## Table 5

| Patient No. | A375 Membranes | Immunoreactivity of Patients | | Clinical Response |
| | | MAC-3 Extract | MAC-1 Extract | |
|---|---|---|---|---|
| 1 | 41, 43, 68, 70 | — | 41, 43, 75, 70 | + |
| 2 | 38, 41, 43, 70 | — | 38, 41, 43 | + |
| 3 | — | — | — | — |
| 4 | 43, 65, 78 | — | 60 (faint) | — |
| 5 | 38, 41, 43, 68, 70 | — | 38, 41, 43, 55 75 | + |
| 6 | 36, 38, 41, 43, 68, 70 | — | 38, 41, 43, 80 | ? |
| 7 | 43 faint | — | — | — |
| 8 | 26, 28, 38, 41, 43, 68, 70 | — | 38, 41, 43, 80 | ? |
| 9 | — | — | — | — |
| 10 | 45 (faint) | — | — | — |
| 11 | — | — | 43 (faint) | ? |
| 12-15 | — | — | — | ? |

EP 0 308 265 A2

As indicated in the above Table 5, six out of eight patients receiving MAC-1 developed antibodies primarily to 38 kd, 43 kd and 70 kd RAAs and two of these patients showed tumor regression. An additional patient (#5) showed regression when he was subsequently treated with interleukin 2. None of the patients immunized with MAC-3 developed antibodies reactive with any of the RAAs except for faint reactivities seen by patients # 10 and # 11 to single antigen bands; it should be noted that such weak reactivities were also detectable with sera from these patients prior to their immunizations. The positive clinical responses seen in a few patients treated with MAC-3 may have been caused by the adjuvant since the responses were generally observed within days or weeks after immunotherapy was initiated, a time earlier than that usually required for the development of specific RAAbs.

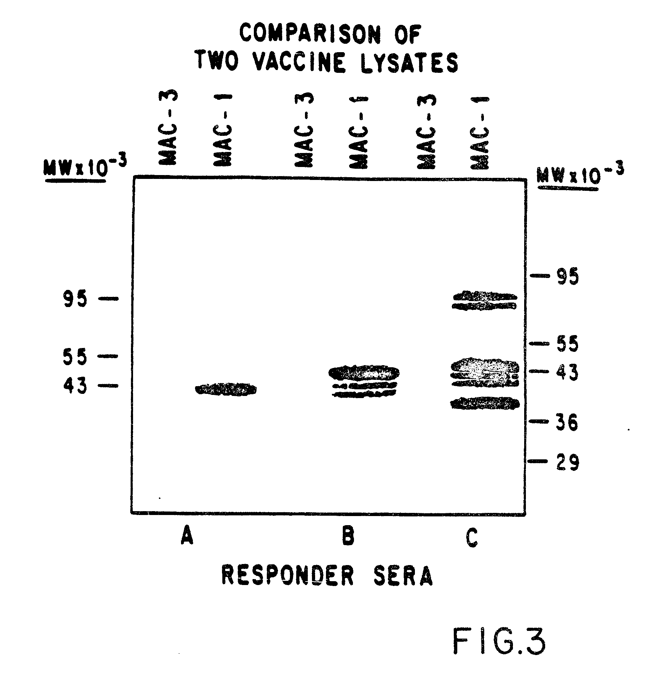

FIG. 3 illustrates the results of three immunoblots in which RAAbs from three different ILI patients (selected from responders in Table 2) were allowed to react with antigens in MAC-1 or MAC-3 extracts. The major reactivity observed with each antiserum was with 38 kd and 43 kd RAAs in MAC-1 alone. Molecular weight (MW) markers on the left were used for the Western blot with antiserum A. The two Western blots (MAC-3, MAC-1) with antisera B and C have as MW references those on the right side (near C).

These results may be compared with FIG. 1 in which the reactivity of antibodies in post-immune sera from Patient No. 2 with the 43 kd, 38 kd and 70 kd RAAs from ING-A cells is illustrated. This patient responded clinically to subcutaneous immunizations with the MAC-1 lysate preparation.

When the ING-A cell lysate is prepared according to the protocol used for preparing MAC-1 lysate, RAAs are localized in the insoluble fraction obtained as a pellet by centrifugation at 10,000 X g for 15 minutes. The soluble supernatant is free of RAAs as assessed by immunoblot analysis.

Example 2.

Characterization of RAAs

2(a) Size on SDS-polyacrylamidge gel electrophoresis.

The specific RAAs of this invention are distinguishable from one another and also from other antigens associated with tumors and cells derived from tumors based upon their sizes and immunological properties. Carcinoembryonic antigen (CEA) is much larger than any of the RAAs, as are several of the antigenic determinants which may be identified with existing murine monoclonal antibodies. Human chorionic gonadotropin (HCG) consists of two subunits both of which are distinguishable from the RAAs described in a reducing SDS-PAGE system (the $\beta$ subunit of HCG is 35 kd in size and its $\alpha$ subunit is 16 kd in size [Pierce et al., Ann. Rev. Biochem., 50, 465-495 (1981)]).

There is a rapidly growing list of tumor-specific antigens identified with murine monoclonal antibodies. The available information distinguishes these antigens from RAAs of this invention on the basis of size or relatedness to normal cellular constituents (Monoclonal Antibodies and Cancer Therapy, Reisfeld et al., eds., supra). The availability of monoclonal antibodies has lead to the detection of a 92 kd, a 23 kd, and a 17 kd antigens in urinary bladder cancer, Ben-Aissa et al., Br. J. Cancer, 52, 65-72 (1985). However, these monoclonals do not react with melanoma cells (the principal source of the 19 kd to 23 kd RAA described herein).

A monoclonal antibody specific for a 43 kd surface protein of human leukemia cell line (THP-1) cross-reacts with the intermediate filament vimentin found in normal cells. Hermal et al., J. Cell Sci,. 73, 87-103 (1985). A 52 kd protein is released by human breast cancer cells [Capony et al., Biochem. Biophys. Res. Commun., 108, 8-15 (1982)], and a high molecular weight glycoprotein (220 kd to 400 kd) as well as a 90 kd protein are found in membranes of human breast cancer [Schlom et al., Cancer, 54, 2777-2794 (1984)]. A sarcoma-specific 70 kd antigen appears to be different from the 68 kd to 70 kd RAA of this invention in that the sarcoma 70 kd antigen was not detectable in carcinoma cell lines [Feit et al., Cancer Res., 44, 5752-5756, (1984)].

Metabolic labeling of ING-A (A375) cells with $^{35}$S-methionine. A375 cells were grown in 100 mm petri dishes to 70% confluency and were incubated with 10 ml of serum-free RPMI medium followed by fresh methionine-deficient media containing 3 μl of $^{35}$S-methionine (1148 Ci/nmol, 1 mCi/0.077 ml) (NEN Research Products, Boston, Massachusetts). Both cells and media were harvested at 1, 2, 4.5, 22 and 28 hours after exposure to $^{35}$S-methionine.

Antigens were immunoprecipitated by treating $^{35}$S-methione-labelled extracts of A375 cells with protein-A sepharose-bound IgG from a responding patient. The antigens were visualized by autoradiography following SDS-PAGE of immunoprecipitated antigens [according to the procedure of Sen et al., Proc. Nat'l Acad. Sci. (USA), 80, 1246-1250 (1983)]. Proteins immunoprecipitated from conditioned media and cell extracts by pre-immune IgG bound to protein-A Sepharose® served as controls. Numerous bands, including a doublet around 38 kd and 43 kd molecular weight, appeared in the immunoprecipitation lanes of cell extracts with RAAbs. In contrast, immunoprecipitation of the conditioned media using RAAbs showed one band in the 38 kd region. The appearance of this band at 4.5 hours coincided with the appearance of a 38 kd protein species in the conditioned media at 4.5 hours. This experiment demonstrates that the 38 kd antigen is secreted by the A375 (ING-A) cells.

## 2(b) Partial purification of RAAs from tumor cell extracts.

Cell line A375 is a malignant melanoma cell line described by Todaro and colleagues. Giard et al., J. Nat. Cancer Inst., 51, 1417-1423 (1973). In a typical procedure for the isolation of RAAs, A375 cells growing in roller bottles may be washed and subsequently exposed to extraction buffers containing nonionic and ionic detergents as described above which detergents are capable of solublizing proteins from cell membranes or total cellular proteins. After high speed centrifugation (10,000 X g, 4° C) the soluble supernatant is used for further purification using conventional chromotography steps as well as affinity chromatography (e.g., resins containing immobilized antibodies reactive with regression-associated antigens or lectins with high affinity for these antigens).

Approximately $10^8$ A375 cells growing in roller bottles were rinsed twice with standard phosphate buffered saline (PBS) and then were extracted with a small volume (50 ml) of 0.2% Triton X-100® buffer for 5 min. at room temperature. The solution was removed and debris was pelleted by centrifugation (10,000 X g, 4° C). The supernatant containing RAAs was passed over an antibody affinity column containing immunoglobulins from patient #2 serum containg RAAb's purified on a Zetachrom® membrane filter according to supplier's instructions (AMF Lab Products, Meriden, Connecticut). Ten milligrams of the immunoglobulins were coupled to an Affi-gel 10® resin according to the supplier's instructions (Bio-Rad, Richmond, California). The unbound proteins were washed with 0.02% Triton X-100®, 25 mM Tris-HCl (pH 7.5) and 0.1 M NaCl. The column was then washed with distilled water. The bound RAAs were eluted using 1 M NaCl, 0.1% NP40® (Sigma Chemical Co., St., Louis, Missouri), 0.5% sodium deoxycholate, 0.1% sodium dodecyl sulfate, 25 mM Tris-HCl (pH 7.5), 1% aprotinin. The eluant was dialyzed against 0.01 M ammonium bicarbonate, frozen and lyophilized.

The lyophilized powder was suspended in 0.05% trifluoracetic acid and 4 mg total protein was loaded onto a standard C-8 column for high performance liquid chromatography (Beckman Instruments). Elution was performed using a linear 0 to 100% acetonitrile gradient going from peak 1 (pass through) to peak 10. A sample from each peak was lyophilized and analyzed by immunoblotting using post-ILI serum (1:1000 dilution) from Patient #2 of Table 1. The Coomassie blue-stained SDS-PAGE revealed that greater than 40% of the detectable protein in peak 6 was in species having 43 kd and 70 kd sizes as shown in FIG. 2.

The progress of purification was monitored using the immunoblotting technique to verify the location of specific 68 to 70 kd, 38 kd and 43 kd regression-associated antigens. The degree of purity of products is assessed by determination of protein concentration as well as comparison of total protein pattern in specific fractions containing RAA's with that of the starting material. The total protein patterns of different samples were monitored by staining the SDS-PAGE with Coomassie blue protein stain or with silver staining to provide a more sensitive indication of total proteins. This general scheme has been used to successfully purify the 43 kd and 70 kd antigens from the A375 cell line and has proven effective also for comparable purification from human ovarian and squamous carcinoma cell lines. The general scheme is also expected to permit purification of the 20 kd antigen from A375 cells and of RAA's from all other cell lines and tissue extracts expressing RAA's.

## Example 3

## Isolation of Preparations Containing RAAs

## 3(a) RAAs in particulate fraction tumor cell lines. Preparation of particulate fraction from cells and cell lines.

Six grams wet weight of cultured tumor cells were lysed in approximately four volumes of Buffer L [50 mM sodium phosphate (pH 7.2) containing 5 mM EDTA and 5 mM EGTA] by five strokes of homogenization in a motor driven Teflon® homogenizer. The cell lysate was clarified at 3,000 X g for 10 minutes, the supernatant was saved and the pellet was suspended in three volumes of Buffer L by homogenization. This suspension was centrifuged again at 3,000 X g for 10 minutes. The supernatant was saved and the pellet was once more resuspended in three volumes of Buffer L and clarified by centrifugation as above. All three supernatants were pooled and were centrifuged at 100,000 X g for 1 hour.

The pelleted particulate preparation was resuspended in Buffer L supplemented with 50% sucrose by five passages in the motorized homogenizer. A ten milliliter sample of the suspension was layered on the bottom of a 40 ml VTi50 centrifuge tube, (Beckman Instruments, Irvine, California) containing a discontinuous gradient of 50%, 40%, 30% and 20% sucrose. Tubes were sealed and spun in VTi50 rotor (Beckman Instruments) for 2 hours at 45,000 rpm (with slow acceleration and no application of the brake).

Particulate material RAAs banded at approximately 40% sucrose. This band was harvested by direct aspiration, diluted five-fold with 10 mM phosphate buffer, pH 7.2 and centrifuged at 100,000 X g for one hour. Pelleted particulate material containing the antigens are resuspended by homogenization in 3 ml of 10 mM phosphate buffer, pH 7.2 and stored frozen at -20°C. In a typical preparation protocol approximately 6 mg of total protein was present in a final particulate material pellet, while approximately 190 mg of protein was discarded at various stages in purification starting which 6 g wet weight of cells.

3(b) RAAs from conditioned media of cultured tumor cells. Purification of secreted regression-associated antigens.

In view of the localization of the 20 kd, 38 kd, 43 kd and 70 kd RAAs in the cell particulate fraction, it was of interest to assess whether any of these antigens could be recovered in serum-free media obtained from cultured tumor cells. Preliminary immunoblot experiments indicated that the serum-free "conditioned" media obtained from confluent cultures of A375 cells or 69-2 cell contain RAAs with the 38 kd antigen in abundance. In FIG. 1, the lanes numbered 2, contained the conditioned media RAA proteins from ING-A (A375) cell cultures.

Purification of the 38 kd antigen secreted by both A375 and 69-2 cells into the culture medium was performed as follows. Two liters of medium was conditioned, for 48 hours by 3 X 10$^9$ A375 cells, centrifuged at 10,000 X g for 10 minutes, and then filtered through a 0.45 μ Nalgene® filter (Nalgene, Inc. Rochester, New York). The filtrate was concentrated to 50 ml using an Amicon YM10 filter (Amicon Corporation, Danvers, Massachusetts) and dialyzed against two liters of Buffer A [10 mM NaPO$_4$ (pH 7.4)], followed by centrifugation at 10,000 X g for 20 minutes. The supernatant from the centrifugation step was loaded onto a DEAE-Sephacel column (Pharmacia, Uppsala, Sweden) equilibrated with buffer A. The column was then washed with 75 ml of buffer A in order to remove unbound material. The 38 kd antigen did not bind to the column and was recovered in the unbound fraction which was concentrated on an Amicon YM10 filter to a volume of 10 ml.

The concentrated DEAE-purified antigen was applied to a heparin-agarose (Sigma Chemical Company, St. Louis. Missouri) column (2 ml) equilibrated with Buffer A. The column was sequentially washed with 20 ml of each of 50 mM, 100 mM, 200 mM and 500 mM NaCl in Buffer A. The 38 kd antigen eluted primarily in 200 mM NaCl wash, but smaller amounts also eluted in the 100 mM and 50 mM washes. The 200 mM eluent was concentrated on an Amicon YM10 filter to 1 ml, applied to a C8 reverse phase HPLC column and eluted with a 30%-60% linear acetonitrile gradient. The 38 kd antigen eluted at about 50% acetonitrile. Very similar elution properties for the 38 kd RAA have been obtained using a C4 reverse phase HPLC column. Analysis of the 38 kd material by reducing SDS-PAGE analysis followed by silver staining showing this 38 kd material to be ≧ 95% pure. In a typical preparation following the procedure described above, approximately 30 μg of 38 kd antigen preparation was obtained from about 300 mg of total starting protein present in 2 liters of media conditioned for 48 hours with 3 X 10$^9$ A375 cells.

Substantial purification of secreted 38 kd RAA was achieved using this procedure. The HPLC-purified antigen represents ten thousand-fold purification over the conditioned media, based on the amount of other contaminating proteins eliminated, as may be gathered from Table 7 in which the degree of purification achieved at various steps in the process as indicated. In Table 7, the term "fold purification" refers to the extent of purification based upon removal of contaminating proteins.

Table 6

Purification of 38 kd Antigen
From Condition Media of A375 Cells

| Step No. | | Volume (ml) | Total Protein (mg/ml) | Protein (mg) | Fold Purification (Approximate) |
|---|---|---|---|---|---|
| 1 | Conditioned media | 1500 | 0.15 | 225 | 1 |
| 2 | DEAE flow-thru concentrated | 10 | 0.19 | 1.9 | 110 |
| 3 | Heparin agaraose (200 mM salt eluent) | 1 | 0.178 | 0.178 | 1100 |
| 4 | HPLC C8 column, acetonitrile eluent | 1 | 0.020 | 0.02 | 9900 |

## Example 4

### Patient Monitoring by Examining Antibodies to RAAs

#### 4(a) Monitoring titers of RAAbs in patients regressing tumors after intralymphatic immunotherapy.

Serum samples from patients undergoing immunotherapy are tested as described above using immunoblotting of various human tumor cell extracts containing RAAs. Various dilutions of sera samples from each patient are evaluated for the ability to detect one or more of the specific size RAAs.

RAAbs have thus been quantitated in sera obtained at different times after initiating a therapy in a patient with metastatic ovarian cancer. Approximately six weeks after initiation of immunotherapy, one patient's serum had an RAAb titer of about 1:2000 against the 43 kd RAA and this titer reached 1:5000 three months later. Similar demonstrable elevations in titers of RAAbs to one or more RAA have been observed in patients as their tumors begin to regress subsequent to intralymphatic treatment of various other malignancies.

The assay presented here for the quantitation of RAAb titer may also be used in monitoring the effects of drugs on patients undergoing immunotherapy. This is illustrated by results obtained with a thyroid cancer patient who demonstrated tumor regression after intralymphatic immunotherapy but was subsequently treated with a high dose of prednisone for symptoms of shortness of breath. Such steroid therapy is known to suppress the immune system. With 5 days after initiating high dose prednisone therapy, there was complete elimination of detectable RAAb titer against the 70 kd RAA as compared to a titer of 1:2000 detected previously in the same patient. Thus, the formation of RAAbs appears to follow a normal course of an active immune response to an antigen in that the RAAb titer is suppressed by immunosuppressants such as prednisone.

#### 4(b) Monitoring the RAA specificities of RAAbs in patients undergoing intralymphatic immunotherapy.

The techniques according to the present invention are useful in the assessment of response to immunotherapy not only for determining the titers of RAAbs but also for determining the different molecular species of the RAAs to which the RAAbs are directed. Different reactivities develop during the course of immunotherapy, as demonstrated by results for one patient with an initial reactivity directed against the 43 kd antigen followed by the appearance of RAAbs reacting with this 43 kd species as well as with the 38 kd and the 70 kd RAAs. The detection of RAAbs correlates in this patient with the complete regression of pulmonary metastasis of an ovarian adenocarcinoma. Similarly, in a second patient the detection of RAAbs to the 43 kd nd the 70 kd RAAs coincided with an evaluable reduction in the size of a pulmonary metastasis of thyroid cancer as well as a profound improvement in the patient's sense of well-being, physical stamina and appetite.

## Example 5

### Development Of Antibodies Against Human RAAs in Animals

#### 5(a) Preparation of High Titer Polyclonal RAAbs in Rabbits.

Antisera may be specifically produced by immunizing rabbits with injections of purified RAAs according to the present invention as follows. A first inoculation may contain membranes of soluble RAAs (purified from solubilized membranes or from conditioned medium) with Freund's incomplete adjuvant or with alum-adsorbed tetanus toxin as an adjuvant. Succeeding inoculations may contain the RAA and Freund's incomplete adjuvant. The animals are bled to obtain sera. Polyclonal antibodies may be isolated from the sera by conventional techniques known in the art, Handbook of Experimental Immunology, Vol. 3, Weir, ed., A 3.1 to A 4.10, Blackwell Scientific Publications (1978). Alternately, affinity columns containing purified RAAs bound to Affi-gel 10® (Bio-Rad) may be prepared using supplier's instructions. Highly specific polyclonal antibodies may be prepared using this affinity column by conventional procedures. Affinity Chromatography, 41-44 and 92-95 Pharmacia AB, Uppsala, Sweden. High-titer (i.e., greater than 1:10,000) rabbit antisera specific for 38 kd, 43 kd and 70 kd human RAAs have been produced, as indicated in FIG. 1. with sera from rabbit 209.

5(b) Monoclonal antibodies.

Monoclonal antibodies according to the present invention may be produced according to the procedure of Kohler et al., Nature, 256, 495 (1975) with the substitution of a preparation of RAA according to Example 4 for an antigen employed therein. Kohler et al. is incorporated by reference herein.

Basically, monoclonal antibodies are produced by injecting mice with immunizing doses of RAAs, as described above for rabbit immunization. Spleens are removed from the immunized animals, and spleen cells are fused to myeloma cells using a fusogen, such as polyethylene glycol. Hybridoma cells producing monoclonals are selected for in a selective growth medium such as the conventional HAT medium. Monoclonal antibodies specific for RAAs may be isolated by chromatography from media in which such hybridomas have been cultured, Brown et al., J. Immunol., 131, 180-185 (1981).

Example 6

Use of Antibodies Directed Against Regression-Associated Antigens

6(a) Regression-associated antigen purification.

Affinity chromatography using RAAbs bound to appropriate matrices such as Sepharose® or Affi-gel® can be used to generate highly-purified preparations of RAAs from human tumor cell extracts. Example 2(b) illustrates the successful isolation of RAAs from A375 cells using RAAb affinity chromatography as one of the purification steps.

6(b) RAA preparation for cancer immunotherapy.

Since high titer human and rabbit RAAbs (Examples 1 and 5) react with specific antigens in fresh tumor-derived extracts, these antibodies offer a powerful means for the isolation of RAAs from a patient's own tumor, or tumor cell line. Extracts of gram quantities of the tumor or tumor cell line, where available, may be prepared in the cell lysis buffer described by Sen et al., Proc. Natl. Acad. Sci. (USA), 80, 1246-1250, (1983) and subsequently diluted prior to loading onto affinity columns of Sepharose coupled to high titer rabbit or human RAAbs (purified IgG fractions). Proteins specifically bound can be eluted from the columns by dissociative buffers and characterized by immunoblotting using known RAAbs and those preparations reacting positively with one or more of the RAAbs may be employed to actively immunize the given patient as part of a therapeutic regimen.

6(c) Drugs may be targeted to a tumor according to the present invention.

An anti-cancer drug may be bound to a monoclonal antibody against an RAA (i.e., a monoclonal RAAb) of the sort described in Example 6. Such antibody-mediated drug delivery systems are reviewed in Rodwell et al., Biotechnology, 3, 889-894 (1985) which is incorporated by reference herein.

By introducing a monoclonal antibody specific for RAAs linked to an anticancer drug into a bodily fluid (blood, lymph or any other appropriate fluid such as cerebrospinal, etc.) of a patient, such a drug may be selectively delivered to tumor cells expressing an RAA for which the monoclonal RAAb is specific. It is anticipated that such binding of a tumor cell with an anticancer drug will preferentially exert an adverse effect on its survival.

Diagnostic in vivo imaging of malignancies using high titer polyclonal or monoclonal RAAbs coupled to as radioisotope (e.g. $I^{131}$, $^{90}Y$, $^{111}In$) or heavy metal (e.g., albumin-coated magnetite, $Fe_3O_4$) may be achieved using radioisotope or magnetic resonance scanning after administration to the patient of the coupled RAAb [Lauterbur, P.C., 47-57, in Accomplishments in Cancer Research 1985, Fortner et al., eds., J. B. Lippincott Co., 1986; Weinstein et al., pages 473-487, in Monoclonal Antibodies and Cancer Therapy, Reisfeld and Sell, editors, A.R. Liss, Inc., New York 1985].

Example 7

Use Of RAAs in Animal Model Systems to Examine Efficacy As Active Immunogens

Nine white male New Zealand rabbits were subcutaneously immunized in their footpads, three rabbits with membranes from 69-2 cells prepared as described in Example 3, three rabbits with membranes from A375 cells prepared as described in Example 3, and three rabbits with 150 μg per rabbit (3 rabbits) of the DEAE-Sephacel flow through fraction of conditioned medium as described in Example 3. Five untreated rabbits of the same type served as controls. Immunized rabbits were boosted twice prior to tumor inoculation. All rabbits were then challenged with equal amounts (5 x 10⁶ viable cells each) of a rabbit squamous cell carcinoma. Tumors developed in the control rabbits and progressed rapidly leading to the death of the animals in 4 to 6 weeks. All immunized rabbits developed small tumors with extensive tumor necrosis, and all survived at least two months. Tumors completely regressed by 3 months in 6 of the 9 immunized rabbits (one from each group of three immunized rabbits failed to show tumor regression). Antibody responses in the immunized rabbits were detected by immunoblotting and found to be very similar to those observed in responding patients as in Examples 1 and 2, and also reacted with RAAs in fresh extracts of human melanoma and breast cancers and rabbit squamous cell carcinoma.

Example 8

Quantitation of RAAs to Monitor Tumors

A "Dot Blot" Immuno Assay for RAAs.

A grid of 1.5 cm X 1.5 cm squares are drawn on 0.45 micron pore size nitrocellulose filter paper (Schleicher and Schuell, Inc., Keene, New York). The paper is rinsed in distilled water for 5 minutes and allowed to air dry. A 20 μl sample of a test extract presumed to contain an unknown amount of RAAs is mixed with an equal volume of 0.05M Tris HCl (pH 7.4) 0.28 M NaCl, 1.4% Triton X-100®, and 0.2% SDS, and heated to 100°C for 5 minutes. The sample is centrifuged at 10,000 X g and the supernatant is spotted with a micropipet within a square on the nitrocellulose filter. The filter is dried and then fixed for 15 minutes in a solvent containing 10% acetic acid and 25% isopropanol with constant agitation; this is followed by several rinses in water. The filter is then processed according to the immunoblotting technique described in Example 1, with the exception that a one hour incubation with goat anti-human antibody conjugated to horse radish peroxidase (1:2000) is substituted for the incubation with radioiodinated Staphylococcus protein A. After this one hour incubation and washing according to the immunoblot procedure, the individual squares of nitrocellulose are cut out and placed into individual wells of a multiwell plate (Costar, Cambridge, Massachusetts). Staining solution is added to each well in the amount of 0.5 ml of phosphate-buffered saline or isotonic citrate buffer (pH7) containing 0.6 mg/ml or o-phenylenediamine dihydrochloride supplemented with 1 μl of 30% hydrogen peroxide. The solution with the filter square is incubated in the dark for 30 minutes and color formation is stopped by adding 0.5 ml 4 N H₂SO₄ per well. Absorbance is then measured in a spectrophotometer at 490 nm. This assay provides a linear quantitation of RAAs when increasing amounts of A375 membranes are spotted onto the nitrocellulose filter paper.

Example 9

NH₂-Terminal and Internal Amino-Acid Sequence Analysis of 38kd Antigen

Mycoplasma hyorhinis (ATCC No. 23839) was cultured in a medium containing the following ingredients: 2.1% PPLO (DIFCO Laboratories, Detroit, Michigan) broth, 0.25% yeast extract, 0.5% glucose, 20% fetal calf serum, 100 units/ml penicillin, 100 μg/ml streptomycin. Twenty-five milligrams of M. hyorhinis protein was harvested by centrifugation as a washed bacterial pellet, and membrane bound hydrophobic proteins were isolated by solubilization of M. hyorhinis with 4 ml of phosphate buffered saline containing 2% Triton X-114® for 4 hours at 4°C.

Insoluble components were removed by centrifugation at 4°C for 10 minutes at 10,000 x g, and TX-114®

soluble material was incubated for 5 minutes at 37°C to induce condensation of Triton X-114. The resulting cloudy suspension was centrifuged at 10,000 x g for 10 minutes at 22°C. The aqueous phase was removed by aspiration, the detergent phase was brought to the original volume with PBS, and the phase separation procedure, as described above was repeated. The TX-114® phase contained the 38 kd, 43 kd, and 68kd antigens according to the present invention as determined by immunoblotting as described in Example 8. Three volumes of ethanol were added to the Triton extract at 4°C to precipitate the proteins and wash away the Triton X-114.

The sample was then centrifuged at 10,000 x g for 10 minutes at 4°C and the resulting pellet was dissolved in SDS containing sample buffer, and the resulting solution was loaded onto a 10% SDS-PAGE gel and electrophoresed. The proteins were then transferred from the SDS-PAGE gel onto activated GF/C filters (Whatman). The conditions for SDS-PAGE and electroblotting were essentially the same as described in J. Vandekerckhove et al., Eur. J. Biochem., 152, 9-19 (1985) and Applied Biosystems User Bulletin No. 25, November 18, 1986. The proteins on the electroblot were visualized by staining with a dipentyl-oxacarbocyanine iodide, and the 38 kd band was excised and was sequenced on an Applied Biosystems 470A sequencer. The following N-terminal sequence was determined: Thr-Ser-Asn-Thr-Gly-Val-Val-Lys-Gln-Glu-Asp-Val-Ser.

Following N-terminal sequence analysis, the 38 kd protein in the electroblots was treated with CNBr (10µl of 0.1 g/ml) in 100 µl of 70% formic acid for 16 hours at room temperature to open new amino terminii following methionine residues for sequence analysis. The following sequence, wherein "X" denotes an unidentified amino acid and a "/" denotes an alternative identification for an amino acid, was obtained from the 38kd blots after CNBr cleavage: X-X-X-Tyr/Leu-Phe-Val-Thr-Val/Asp/Asn-Glu-Ileu-Leu-Tyr-Asp-Val-Gly-Val-Phe.

## Example 10

## N-Terminal Sequence Analysis of 43kd Antigen

TX-114® extraction was performed on 50 mg of M. hyorhinis protein harvested according to the procedure described in Example 9 above. The TX-114® extract was made up to 10 ml in 20 mM ethanolamine, pH 9.0 and loaded onto a 1 ml column of DEAE-Sephacel equilibrated with the same buffer containing 0.5% Thesit® (Boehringer Mannheim GmBH, W. Germany) The proteins were eluted from the column using 20 mM sodium phosphate (pH 7.2) and 50 mM NaCl. The DEAE-Sephacel column was not used to enrich the antigens, but rather was primarily used to exchange the proteins from TX-114® into Thesit®. The eluted material from the above step was loaded onto 1 ml of heparin-agarose in a column (Sigma Chemical Company, St. Louis, Missouri) which was equilibrated with 20 mM sodium phosphate (pH 7.2) containing 50 mM NaCl.

The heparin-agarose flow-through contained 43 kd antigen, reduced levels of 38 kd antigens and several other proteins. A large portion of the 38 kd antigen was bound to the column and was eluted with 5 ml of 250 mM NaCl in 20 mM sodium phosphate (pH 7.2). The heparin-agarose flow-through was adjusted to 150 mM NaCl and loaded onto a 1 ml hydroxyapatite column (Bio-Rad Laboratories). The 43 kd antigen flows through and is mostly removed from contaminating bands around the same molecular weight. The 43 kd antigen which flowed through both of the above columns was concentrated to 150 µl using a YM10 filter (Centricon, obtained from Amcion Division, W.R. Grace and Company, Daneers, Massachusetts). The 43 kd antigen was electroblotted onto GF/C filters and the following N-terminal sequence, in which a "/" indicates an alternative identification for an amino acid, was obtained following the procedure as described in Example 9 above: Ser-Gly-Glu-Thr-Asp-Lys-Glu-Gly-Lys-Ile-Arg-Phe-Asp-Asn-Cys/Ser-Phe-Val-Lys-Asp.

## Example 11

## Genomic Clone of the M. hyorhinis Gene Sequence Encoding the 38 kd Protein

From the amino acid sequence obtained from a CNBr-treated sample of gel-purified 38 kd protein, a mixed-sequence oligonucleotide probe (an 18 mer) was chemically synthesized according to the instructions of the inventors by Synthetic Genetics, San Diego, California for: TAYGAYGTNGGNGTNTGG. All possible probes were constructed according to this sequence wherein "Y" is a pyrimidine, "R" is a purine and "N" is any nucleotide residue. The last two G residues were incorrect and were predicted based upon an error in the amino acid sequence data.

Southern blotting experiments showed that this probe hybridized to a 3.4 kb HindIII fragment of M. hyorhinis DNA. Therefore, HindIII DNA fragments migrating at the 3.4 kb size range were electro-eluted from agarose gel and ligated to the plasmid vector, pUC19 [Biolabs, Beverly, Massachusetts] and used to transform E. coli cells to create a library of 3.4 kb M. hyorhinis DNA HindIII inserts. On screening the 3.4 kb HindIII-fragment library with the 18 mer probe, a clone designated pM38-29/3, was isolated. It contained the 18 mer probe sequence identified by brackets in FIG. 4, but this clone was not complete. A 26 mer probe sequence identified by brackets in FIG. 4 was also used for screening. A more complete sequence was determined and is illustrated in FIG. 4.

In FIG. 4, it is believed that a complete amino acid sequence for a 38 kd RAA is provided in which residues indicated by an asterisk ("*") are hydrophilic, residues indicated by underlining are hydrophobic, and in which potential glycosylation sites (Asn-X-Ser/Thr) are overlined. Hydrophilicity /hydrophobicity data and glycosylation data may be used to construst immunogenic fragments and peptides and to construct analogs of the 38 kd polypeptide.

The nucleotide sequence of a 400 bp AluI fragment obtained from clone pM38-29/3 containing the 18 mer sequence was determined [according to the procedures of Sanger et al., Proc. Nat'l. Acad. Sci. (USA), 74, 5463 (1977)]. It was found that the triplet TGA, which may code for termination in E. coli, codes for trp in M. hyorhinis, as indicated by parentheses in FIG. 4. The 400 bp AluI fragment was then used as a probe to screen a library generated by cloning 11 kb EcoRI DNA fragments of M. hyorhinis in pUC19 [Maniatis et al., Molecular Cloning, Cold Spring Harbor Laboratory, Cold Spring Harbor, New York (1982)]. The 400 bp AluI probe hybridized to an 11 kb EcoRI fragment of M. hyorhinis DNA. A clone pM38-EI-1 was isolated. The insert was found to contain a 4.4 kb insert with 6.6 kb of the above 11 kb M. hyorhinis DNA sequence being deleted. This clone contained sequences that hybridized to a mixed-sequence probe: AAYACNGGGNGTNGTNAARCAR-GARGA, predicted from the N-terminal amino acid sequence of the 38 kd protein. All possible probes were constructed for the oligonucleotide sequence indicated above wherein "Y" is a pyrimidine, "R" is a purine and "N" is any nucleotide base. In FIG. 5, the restriction map of this 4.4 Kb is illustrated. The indicated restriction endonuclease cleavage sites indicated by small, vertical bars in FIG. 5 in the map of clone pM38 EI-1 matched with corresponding sites which were those independently determined through restriction endonuclease analysis of M. hyorhinis DNA.

Example 12

Genomic Clone of the M. hyorhinis Gene Sequence Encoding the 43 kd Protein

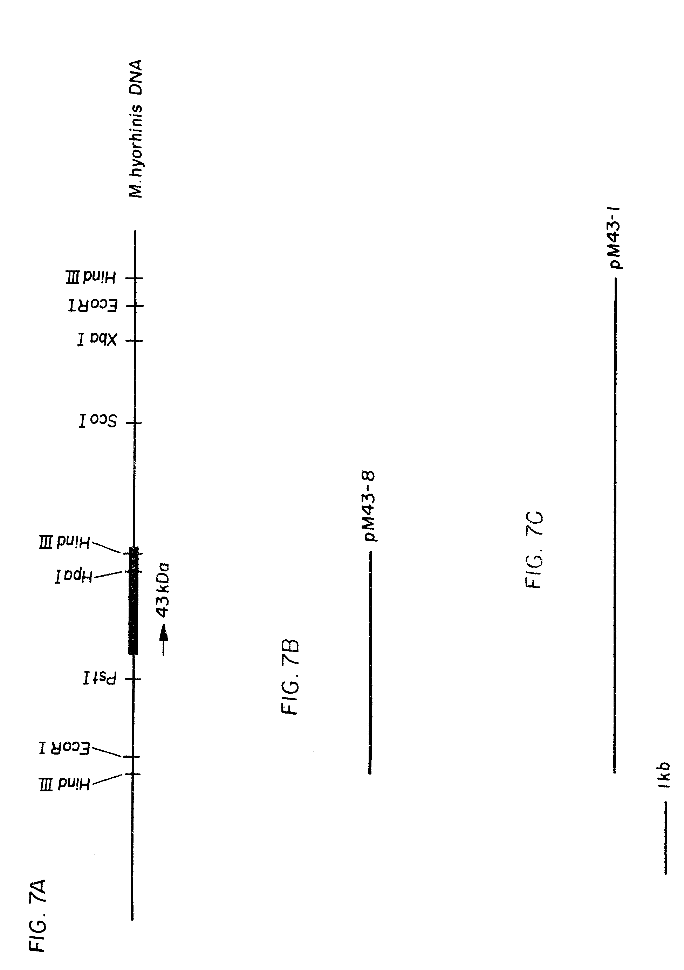

From the N-terminus amino acid sequence of the 43 kd protein (see Example 10) a 44 mer oligonucleotide with the following sequence was synthesized: GG(A/T) GAAAC(A/T)GATAAAGAAGG(A/T)AT(A/T)AGAAT(A/T)TTYGATAA. Choices of the codons were made based on the codon usage in the gene encoding the 38 kd protein in mycoplasma. The oligonucleotide was then used to screen a size-selected partially-digested HindIII M. hyorhinis genomic library. The library was constructed by (i) first under-digesting M. hyorhinis DNA with HindIII, (ii) isolating DNA fragments of about 10kb using a sucrose gradient, and (iii) inserting the DNA fragments into the plasmid vector pUC19. Four clones were screened as positive with the oligonucleotide probe described above, and one, pM43-1, was shown to contain the entire coding sequences for the 43 kd protein. The restriction map of the insert is shown in FIG.7, and the map was verified by Southern blot analysis of genomic DNA.

Sequencing of the gene was carried out using standard technology (see Example 11) by both subcloning fragments into M13 and using the primer-directed method. The nucleotide sequence so determined is shown in FIG. 6. There are four TGA codons that appear to code for trp residues in M. hyorhinis.

Example 13

Murine Hybridomas Secreting IgM Antibodies Specific For Regression-Associated Antigens

Two adult female C3H mice were immunized with about 10.0 μg protein aliquots of A375 membrane preparations prepared essentially according to the procedure of Example 3A and was determined to contain regression-associated antigens by immunoblotting as described above. The mice were immunized subcutaneously with membranes admixed with incomplete Freund's adjuvant (total volume 0.2 ml) on days 0, 14 and 21. Serum taken on day 35 revealed an antibody titer of 1:200 against regression associated antigens in the immunoblot assay of Example 8 using A375 (ING-A) membranes as the source of regression associated antigens.

At approximately seven weeks after immunizations commenced, the mice were sacrificed, their spleens were removed and splenocytes were fused to murine myeloma partner cells essentially as described in Kohler et al., supra. Approximately 70 to 80 hybridoma colonies were analyzed. The supernatant media from these colonies were screened for the presence antibodies reactive with A375 cells (ING-A) (10,000 cells per well of a microtiter plate) by an ELISA.

In an ELISA according to the present invention, cultures of A375 cells contaminated with M. hyorhinis, namely ING-A cells, or A375 cells which were free from M. hyorhinis were propagated in RPMI 1640 medium containing 10% fetal calf serum. When the cultures reached confluency, their medium was decanted, cells were rinsed twice with phosphate buffered saline (PBS) and, finally, cells were removed by incubation with PBS containing 2.5 mM EDTA. The cells were pelleted by centrifugation and rinsed once with PBS followed by pelleting and resuspension in PBS at 10,000 cells per 50 μl.

Polyvinyl ELISA plates were prepared with unfixed cells by aliquoting the cell suspensions into 96 well plates using 50 μl per well. The plates were incubated at 37° C overnight to allow for complete drying of the wells and effective attachment of cells to the bottoms of the wells.

The multiwell plates thus prepared were blocked with 5% nonfat milk in PBS. After washing with PBS, individual wells were incubated with 5 μl of hybridoma supernatant diluted with 45 μl of PBS for 3 hours at 37° C. Wells were then washed and incubated with a 1:350 dilution of peroxidase-congugated goat anti-mouse antibody (Sigma Chemical Company) as recommended by the manufacturer. Plates were subsequently washed, incubated with orthophenylenediamine (Sigma Chemical Company) and color formation was terminated by adding 10 μl of 6 N HCl per well. ELISA plates were read at 492 mu and positive signals ranged from 0.7 to 1.3 with background signals of 0.1 to 0.2.

Only antibodies reactive (at a level of greater than 5 to 10 times background signal) with ING-A cells, which contain RAAs, and non-reactive with RAA-negative cells were subjected to subcloning. Approximately six independent subclones were generated in this fashion. The subcloned hybridomas retained their specific reactivities for cells containing RAAs.

In addition, these subclones were screened by the above ELISA for reactivity with a partially-purified heparin-agarose column fraction (250 mM NaCl eluate as described in Example 10) of solubilized regression-associated antigens from M. hyorhinis. These subclones demonstrated some differences in the extent of reactivity with the partially-purified regression associated antigen protein (1/2μg/well). Most had positive titers (3 to 5 times background signals). The clones were designated 3C3, 2H8, 2C8, 3G2, and 2H7. The clone designated 3C3 was deposited on September 16, 1987 as ATCC Deposit No. HB 9540 with the American Type Culture Collection, 12301 Parklawn Drive, Rockville, Maryland 20852.

Typing of these hybridomas determined using an isotyping kit and procedure (available from HyClone Laboratories, Inc., Logan, Utah) revealed them to be secreting IgM antibodies. Preliminary indirect immunoperoxidase or immunofluoresence screening [according to the procedure described in Sheehan et al., Theory and Practice of Histotechnology, eds. C.V. Masby, Co., 310-326 (1980)] on fresh frozen human tumors has shown a monoclonal secreted by the subclone designated 3C3 to be 3+ to 4+ (positive) on two independently-derived Ewing's sarcomas, a mesothelioma, a breast carcinoma metastatic to spleen, lung and liver, and weakly reactive (1+) with a breast adenocarcinoma. This antibody was non-reactive with a biliary carcinoma. Against human tumor cell lines, 3C3-secreted monoclonal antibody reacts strongly with Ewing's sarcoma and mesothelioma cell lines derived from the above tumors as well as osteosarcoma, colon carcinoma, adenocarcinoma of the lung, spindle cell sarcoma, bladder carcinoma and breast carcinoma cell cultures established by enzymatic disruption of minced fresh tumor biopsies followed by plating in RPMI 1640 medium containing between 10% to 20% fetal calf serum. It also reacts with an established tumor cell line designated M14 derived from a human melanoma tumor [Moy et al., J. Surg. Oncol., 29, 112-117 (1985)]. It is non-reactive with two other melanoma cell lines, with human fibroblastic cells in culture and against a panel of frozen sections of normal human tissues (excluding endothelial and myofibroblastic cells) obtained from autopsy of a single patient including esophogous, liver, stomach, skeletal muscle, thyroid, urinary bladder, spleen, pancreas, lung and cerebellum. Certain reactivity of the 3C3 monoclonal antibody has been observed with some human endothelial cells and myofibroblasts in immunohistochemical staining studies using frozen sections of certain tissues.

Example 14

Monospecific Polyclonal Antibodies Reactive With Purified 38 kd and 43 kd Antigens

Polyclonal rabbit sera were generated by immunizing rabbits with the 38 kd RAA protein prepared essentially according to the procedure of Example 9 and isolated from polyacrylamide SDS gels and with the 43 kd RAA protein of Example 10.

Anti-38 kd antibodies were generated by immunization of two rabbits with the protein species migrating at 38 kd in gel slices of preparative SDS-PAGE. The M. hyorhinis protein used for SDS-PAGE was the heparin-agarose bound material ((250 mM NaCl eluant) of Example 9. In Western immunoblot analysis, the anti-38 kd antibody, when used at 1:1000 dilution, recognized the 38 kd antigen in SDS-extracts of M. hyorhinis and other antigens of molecular masses ranging from 36.5 kd to 95 kd in SDS-extracts of several human tumor cell lines and fresh tumor extracts (Table 7). Indirect immunoperoxidase staining with anti-38 kd antibodies revealed positive reactivity to several human primary tumor cell cultures (mesothelioma, osteosarcoma and Ewing's sarcoma) as well as to frozen sections from human mesothelioma and breast carcinoma and also to an M14 human melanoma cell line [Moy et al., supra].

Anti-43 kd antibodies were generated by immunization of two rabbits with 43 kd gel slices from SDS-PAGE of the heparin-agarose flow-through material of Example 10. In Western immunoblot analysis, this anti-43 kd antibody, when used at 1:200 dilution, recognized 43 kd as well as 38 kd antigens in SDS extracts of M. hyorhinis.

Cell cultures identified in Table 7 are cultures derived from primary culutres of the respective human tumors as described under Example 13 or are identified as "ATCC" and are obtained as human tumor cell lines from the American Type Culture Collection (Rockville, Maryland).

## Table 7

## ImmunoReactivity
## of Anti-38kd Sera

| Cell/Tumor Extracts | Protein Bands Detected |
| --- | --- |
| Normal colon tissue extract | (faint) 36.5kd |
| Osteosarcoma cell culture | (strong) 55kd |
| Melanoma cell line (M14) | (faint) 55kd, 68kd |
| Ovarian carcinoma cell culture | (strong) 43kd, (weak) 60kd |
| Metastic colon cancer carcinoma extract | (strong) 55kd |
| Ovarian carcinoma cell culture | (none) |
| Breast carcinoma extract | (faint) 48kd, (faint) 95kd |
| Lymphoma cell | (strong) 43kd, (strong) 60kd |
| Normal fibroblast (G1N-1)/ATCC | (strong) 55kd |
| Colon carcinoma cells (SW4/80)/ATCC | (moderate) 60kd |
| Colon carcinoma cells (LOVO)/ATCC | (moderate) 60kd |
| Lung carcinoma cells (A549)/ATCC | (strong) 58kd, (weak) 60kd |

Example 15

Nucleic Acid Probes for RAA Gene Sequences

Deoxyribonucleic acid hybridization probes may be synthesized using the sequences of FIG. 4 by the procedure of Caruthers. U.S. Patent No. 4,415,732 and ribonucleic acid probes may be made by in vitro transcription from them. Hybridization conditions according to the present invention may generally be defined as reactions functionally equivalent to hybridization carried out in 4 X SSC and 0.5% SDS at a termperature of 65° C in the last wash.

Plasmids including DNA sequences according to the present invention (e.g. as presented in FIG. 5 may be labeled with a radioactive isotope. [Rigby et al., Mol. Biol., 113, 237-251 (1977) or Feinberg et al., Anal. Biochem., 132, 6-13 (1983)] or with a non-radioactive chemical tag [Leary et al., Proc. Nat'l. Acad. Sci. (USA), 80, 4045-4049 (1983)] and used as probes. Such plasmids may also be used to synthesize the labeled RNA probes [Melton et al., Nucleic Acids Res., 12, 7035-7055 (1984)]. The labeled probes may be used to detect the presence of homologous DNA sequences and/or mRNA sequences encoded by these DNA sequences in tumor cells either by the Southern or Northern hybridization procedure [Southern et al., J. Mol. Biol. 98, 503 (1975); Thomas, Proc. Nat'l. Acad. Sci. (USA), 77, 5201-5205 (1980)] or by dot blot or slot blot hybridization [Kafatos et al., Mol. Cell. Biol., 3, 1097-1107 (1983)], or by in situ hybridization techniques [Brahic et al., Proc. Nat'l. Acad. Sci. (USA), 75, 6125-6129 (1978)].

One type of hybridization assay which may be performed using the hybridization probes according to the present invention is called solution hybridization. In this procedure, a labeled probe nucleic acid is added to a solution of a sample to be searched for a target nucleic acid. In order to ensure that both the probe and a target are in a single-stranded state suitable for hybridization, the sample and probe are heated in order to break (denature) the hydrogen bonds which are found between complementary strands of a double-stranded probe or a double-stranded target, or which are found within secondary structure of a probe or target. Upon cooling, the reaction is reversed and double-stranded nucleic acid is allowed to form. The amount of double-stranded nucleic acid which forms may be determined by scintillation counting of the label on the probe after degradation of unhybridized single strands or after isolating double-stranded DNA by passing the hybridization solution over a hydroxyapatite column which selectively retains the double-stranded form.

In another type of hybridization assay to which the probes according to the present invention may be applied, denatured target nucleic acid is immobilized on a support. Retention of a labeled probe on a support-bound target after passage of the support-bound target through a solution containing the probe permits detection and quantitation of the target by measurement of the amount of bound label. See, e.g., Falkow et al., U.S. Patent No. 4,358,535; and Shafritz, European Patent Application No. Al-0062286.

Yet another type of hybridization assay of the present invention in which the probes accordingly may be employed is called a "sandwich" hybridization. A two-step sandwich hybridization procedure involves the use of an immobilized target nucleic acid which is exposed in a first step to a first nucleic acid probe having a first portion complementary to the target and having a second portion which is not complementary to the target. In a second step, a second, labeled nucleic acid probe, which is complementary to the second portion of the first probe, is allowed to hybridize to the first probe, forming a "sandwich" comprising the first probe between the target and the second probe. Dunn et al., Cell, 12, 23-36 (1977).

A one-step sandwich assay may also be performed. This type of assay involves the use of a first nucleic acid probe immobilized on a filter. The first nucleic acid probe immobilized on a filter. The first nucleic acid probe is complementary to a first portion of a target nucleic acid. In a single step the filter-bound first probe is exposed to a sample to be searched for the target nucleic acid sequence and to a second, labeled nucleic acid probe complementary to a second portion of the target nucleic acid which portion is separate from (i.e., non-overlapping with) the portion of the target to which the first probe is complementary. Ranki et al., U.S. Patent No. 4,486,539.

Another approach to hybridization, called blot hybridization involves separating sample nucleic acids according to size by electrophoresis through a gel and then transferring them to a nitrocellulose filter on which they are immobilized in their relative positions on the gel. Because any target in the sample is confined to a distinct band on the filter, even weak signals resulting from small amounts of target may be distinguished from non-specific background after exposure to a radiolabeled probe. Bornkamm et al., Curr. Top. Microbiol. Immunol., 104, 288-298 (1983).

Where a sample is in the form of a touch smear of a fluid, a section through cells, or chromosomal squashes from cells on slides, hybridization may be performed in situ.

Generally, a radioactively labeled probe according to the present invention is applied to the sample which is bound to the slide in a histological preparation. After coating the slide with a photographic emulsion, autoradiographic procedures reveal the location of target-probe hybrids by means of clusters of silver grains formed in the emulsion over the hybridization site.

## Example 16

## Synthetic Peptide Antigens/ Immunogens Related to RAA Sequences

The DNA sequence of FIG. 4 has been used as shown therein to deduce the corresponding protein sequence. Peptides corresponding to different portions of RAA proteins, preferably 12-20 amino acid residues in length, may be chemically synthesized by solid-phase methods [Marglin et al., Ann. Rev. Biochem., 38, 841-866 (1970)]. Such peptides may then be used to elicit specific polyclonal and monoclonal antibodies [Lerner, Nature, 299, 592-596 (1982); Niman et al., Proc. Nat'l. Acad. Sci. (USA), 80, 4949-4953 (1983)]. The DNA sequence provided in FIG. 4 facilitates the design of immunogenic peptides corresponding to different regions of the 38 kd RAA protein, suitable immunogenetic regions of which may be determined according to procedure known to those skilled in the art [Novotny et al., Proc. Nat'l. Acad. Sci. (USA), 83, 226-230 (1986) and Van regenmortel, Trends Biochem. Sci., 11, 36-39 (1986)].

## Example 17

## Production of RAA Antigens or Fragments Thereof Using Recombinant DNA Technology

Complete and partial RAA gene products may be expressed in bacteria, yeast or mammalian expression systems by inserting the DNA sequence of FIG. 4 into plasmid, phage or viral expression vectors [Vieira et al., Gene, 19, 259-268 (1982); Young et al., Proc. Nat'l. Acad. Sci. (USA), 80, 1194-1198 (1983); Bitter et al., Gene, 32, 263-274 (1984); Cepko et al., Cell, 37, 1053-1062 (1984); and Gorman et al., Mol. Cell. Biol., 2, 1044-1051 (1982)]. Alternatively, M. hyorhinis may be cultured, e.g. as in Example 9, and RAAs may be isolated and purified therefrom, e.g., as in Example 9. The expressed proteins may be purified and used in immunotherapy or to raise specific antibodies.

In addition to the ELISA and immunoblot assays described herein, polyclonal and monoclonal antibodies according to the present invention may be used separately or in combination with purified and isolated RAAs according to the present invention in any suitable immunoassay.

Target antigens may be adsorbed to polyvinyl titration plates and various dilutions of polyclonal or monoclonal RAAs may be applied to the individual wells in a radionimmunoassay [Tsu et al., Selected Methods in Cellular Immunology, Mishell et al., eds., Freeman Publishing Company, San Francisco, 373-397 (1980)].

In addition, it is contemplated that immunoassays as described herein may be varied as is clear to one skilled in the art. Such variations include the use of monoclonal and polyspecific antibodies in conventional and sandwich ELISA [Kemeny et al., J. Immunol., Methods, 87, 45-50 (1986).

## Example 18

## Uses of RAAbs

Tumor localization and therapy may be performed employing RAAbs according to the present invention by generally following radiolabeling and scanning procedures set forth in and referenced in Goldenberg, U.S. Patent No. 4,348,376, and Goldenberg, U.S. Patent No. 4,444,744. It is also contemplated that fragments of RAAbs and hybrid chimeric antibodies and fragments of RAAbs are useful for scanning and therapy as well; [Goldenberg, U.S. Patent No. 4,331,647; Stevenson et al., Bioscience Reports, 5, 991-998 (1985)].

Conjugation of RAAbs with immunotoxins may also be employed in therapy. [Blakey et al., BioEssays, 4, 292-297 (198 )].

Although the present invention is described in terms of a preferred embodiment, it is understood that modifications and improvements will occur to those skilled in the art. For example, conventional immunological techniques such radioimmunoassay, immunoprecipitation and ELISA may be suitable for detection of regression-associated antigens.

It is also anticipated that RAAs according to the present invention may be further characterized including amino acid sequencing and convalent modification thereof, if any, of any protein components and oligosaccharide structure determination of glycoprotein components, if any or lipid composition of associated lipid moieties, if any. It is further anticipated that the amino acid sequence of any polypeptides portions of RAAs may be obtained, and a cDNA or a DNA sequence may be obtained therefrom and that antigens of the present invention may be produced using known, recombinant DNA techniques. Furthermore, fragments of RAAs and conjugate analogs and derivatives or fragments thereof of RAAs are also anticipated. Thus, any RAA preparation whether isolated from a cell line or directly from a tumor tissue, whether naturally produced or recombinantly derived, whether possessing the same structure as or the structure of a fragment of an RAA or an analog, derivate, or conjugate of an RAA or a fragment of an RAA disclosed herein are all intended to be encompassed within the scope of the present invention.

It is contemplated that RAAbs and monovalent variants thereof may be produced by recombinant techniques. RAAbs and fragments thereof may also be employed in passive immunization procedures. [Beutler et al., Science, 229, 869-871 (1985)]. Anti-idiotypic antibodies may be raised against RAAbs [Eichmann et al., CRC Crit. Rev. Immunol., 7, 193-227 (1987)] and employed in immunotherapy and purification of RAAbs as described for RAAs above. In particular, RAAbs may be employed in passive immunization therapies as understood by those skilled in the art.

Accordingly, it is intended that the appended claims include all such equivalent variations which come within the scope of the invention as claimed.

The features disclosed in the foregoing description, in the following claims and/or in the accompanying drawings may, both separately and in any combination thereof, be material for realizing the invention in diverse forms thereof.

## Claims

1. A method for detecting the presence of regression-associated antibodies comprising the steps of:
obtaining a first sample of serum from a patient diagnosed as not being in a state of regression and obtaining a second sample from the patient after diagnosis as being in a state of regression;
separately exposing a component of a neoplastic cell to first and to the second samples;
determining the absence of immune complex formation between the component and antibodies in the first serum sample; and
detecting the presence of an immune complex between an antibody in the second sample and the component.

2. A method for monitoring the response of a patient to immunotherapy for symptoms associated with a neoplasm comprising the step of:
determing a circulating level of a regression-associated antibody in a patient.

3. A method for identifying regression associated antigens comprising the steps of:
exposing a component of a neoplastic cell to regression-associated antibodies; and
determining the presence of an immune complex between at least one of the antibodies and the component.

4. A method for purification of regression-associated antigens comprising the steps of:
obtaining a first sample of serum from a patient diagnosed as not being in a state of regression and obtaining a second sample from a patient after diagnosis as being in a state of regression;
disrupting a neoplastic cell into antigenic components of a solution;
chromatographically separating components of the solution into fractions; and
identifying fractions containing regression associated antigens by the presence of immune complex formation of components of a fraction with antibodies in the second sample to regression associated antigens.

5. The method as recited in to claim 4 further comprising the steps of
exposing a component of a neoplastic cell to each of the samples;
determining the absence of immune complex formation between the component and antibodies in the first serum sample; and
detecting the presence of an immune complex between an antibody and the component.

6. A purified and isolated antigen selected from the group consisting of:
regression associated antigens having a molecular weight as determined by reducing SDS polyacrylamide gel electrophoresis within the range of:
about 19,000 to about 23,000 daltons;
about 38,000 to about 45,000 daltons; and
about 65,000 to about 71,000.

37

7. The purified and isolated antigen as recited in claim 6 wherein said antigen does not bind to cationic exchange resins at 50 mM Na$_3$PO$_4$ (pH7) or to heparin agarose resins at 200 mM Na$_3$PO$_4$ (pH 7).

8. A method for performing immunotherapy comprising the step of injecting a patient with an immunogenic amount of an antigen as recited in claim 6.

9. The method for performing immunotherapy as recited in claim 8 wherein the antigen is an antigen according to claim 7.

10. A method for monitoring tumor metastasis comprising the step of determining the level of a regression-associated antigen in a bodily fluid.

11. A monoclonal or monospecific polyclonal antibody exhibiting a specific immunoreactivity with an antigen as recited in claim 6.

12. A method for purifying an antibody as recited in claim 11 comprising the steps of:
contacting a substrate-bound antigen as recited in claim 6 with a solution containing an antibody as recited in claim 11; and
eluting the antibody from the antigen.

13. A drug delivery method for tumor therapy comprising the steps of:
binding a drug to an antibody as recited in claim 11; and
introducing the antibody-bound drug into a bodily fluid of a patient.

14. A method for purfying antigen useful in immunotherapy of cancer patients comprising the steps of:
contacting a substrate-bound antibody as recited in claim 11 with a solution containing an antigen as recited in to claim 6; and
eluting the antigen from the antibody.

15. A method for immunotherapy of tumors comprising the steps of:
obtaining a first sample of serum from a patient diagnosed as not being in a state of regression and obtaining a second sample from a patient after diagnosis as being in a state of regression;
separately exposing a component of a neoplastic cell to the first and second samples;
determining the absence of immune complex formation between the compcnent and antibodies in the first serum sample;
determining the presence of immune complex formation between an antibody and the component; and
introducing the neoplastic cell, or membrane fractions thereof or regression associated antigens isolated therefrom into lymphatic or hematic fluid within a patient.

16. A method for purification of a regression-associated antigen comprising the steps of:
conditioning medium with a cell line secreting a regression-associated antigen having a molecular weight as determined by reducing SDS polyacrylamide gel electrophoresis of about 38 kd;
concentrating protein present in the conditioned medium;
chromatographically separating fractions of the concentrated protein;
concentrating fractions exhibiting appreciable absorbance of 280 nm;
loading the concentrated fractions onto onto a C$_8$ column and eluting with a 0-80% acetonitrile gradient in 0.1% trifluoroacetic acid; and
retaining a fraction eluting at approximately 50% acetonitrile.

17. A purified and isolated regression associated antigen.

18. The antigen as recited in claim 17 wherein said antigen is selected from the group consisting of:
regression associated antigens having a molecular weight as determined by reducing SDS polyacrylamide gel electrophoresis within the range of:
about 19,000 to about 23,000 daltons;
about 38,000 to about 45,000 daltons; and
about 65,000 to about 71,000.

19. The purified and isolated antigen as recited in claim 18 wherein said antigen does not bind to cationic exchange resins at 50 mM Na$_3$PO$_4$ (pH7) or to heparin agarose resins at 200 mM Na$_3$PO$_4$ (pH 7).

20. The antigen as recited in claim 19 whrein said antigen is greater than or equal to 95% pure as determined by reducing SDS PAGE analysis followed by silver staining.

21. The antigen as recited in claim 20 wherein said antigen is encoded by DNA within Mycoplasma hyorhinis.

22. The antigen as recited in claim 20 wherein said antigen is encoded by DNA within a mammalian cell line associated with Mycoplasma hyorhinis.

23. An immunotherapeutic composition comprising:
a purified and isolated regression-associated antigen; and
a pharmaceutically acceptable diluent, adjuvant or carrier.

24. The immunotherapeutic composition as recited in claim 23 wherein said regression-associated antigen is a regression-associated antigen as recited in claim 18.

25. The immunotherapeutic composition as recited in claim 23 wherein said regression-associated antigen is a regression-associated antigen as recited in claim 19.

26. A regression-associated antigen encoded by a purified and isolated nucleic acid described by a nucleotide sequence selected from the group consisting of:
the nucleotide sequence as shown in FIG. 4; a nucleotide sequence which encodes the same sequence of amino acids as encoded by the nucleotide sequence shown in FIG. 4;

a nucleotide sequence which would hybridize with any 20 sequential nucleotides as shown in the nucleotide sequence shown in FIG. 4;

a nucleotide sequence which would hybridize with any 20 sequential nucleotides as shown in the nucleotide sequence shown in FIG. 4 but for the redundancy of the genetic code; and

a nucleotide sequence which encodes an epitope encoded by 18 sequential nucleotides in the nucleotide sequence shown in FIG. 4.

27. A purified and isolated nucleic acid described by a nucleotide sequence selected from the group consisting of:

the nucleotide sequence as shown in FIG. 4;

a nucleotide sequence which encodes the same sequence of amino acids as encoded by the nucleotide sequence in FIG. 4;

a nucleotide sequence which hybridizes with any 20 sequential nucleotides as shown in the nucleotide sequence shown in FIG. 4;

a nucleotide sequence which would hybridize with any 20 sequential nucleotides as shown in the nucleotide sequence shown in FIG. 4 but for the redundancy of the genetic code; and

a nucleotide sequence which encodes an epitope encoded by 18 sequential nucleotides in the nucleotide sequence shown in FIG. 4.

28. A cell transformed with a nucleic acid as recited in claim 27.

29. An expression product of a cell as recited in claim 28.

30. A purified and isolated nucleic acid described by a restriction map shown in FIG. 5.

31. A cell transformed with a nucleic acid as recited in claim 30.

32. An expression product of a cell as recited in claim 31.

33. A monoclonal or monospecific polyclonal antibody exhibiting a specific immunoreactivity with an antigen as recited in claim 26.

34. A monoclonal or monospecific polyclonal antibody exhibiting a specific immunoreactivity with an expression product as recited in claim 39.

35. A monoclonal or monospecific polyclonal antibody exhibiting a specific immunoreactivity with an expression product as recited in claim 32.

36. A method of in vivo imaging comprising the steps of:

injecting an antibody as recited in claim 33 into a patient, said antibody being bound to a radioisotope; and scanning the patient using radioisotope scanning.

37. A method of in vivo imaging comprising the steps of:

injecting an antibody as recited in claim 33 into a patient, said antibody being bound to a heavy metal; and scanning the patient using magnetic resonance scanning.

38. A method of in vivo imaging comprising the steps of:

injecting an antibody as recited in claim 34 into a patient, said antibody being bound to a radioisotope; and scanning the patient using radioisotope scanning.

39. A method of in vivo imaging comprising the steps of:

injecting an antibody as recited in claim 34 into a patient, said antibody being bound to a heavy metal; and scanning the patient using magnetic resonance scanning.

40. A method of in vivo imaging comprising the steps of

injecting an antibody as recited in claim 35 into a patient, said antibody being bound to a radioisotope; and scanning the patient using radioisotope scanning.

41. A method of in vivo imaging comprising the steps of:

injecting an antibody as recited in claim 35 into a patient, said antibody being bound to a heavy metal; and scanning the patient using magnetic resonance scanning.

42. A method for isolating a regression-assocaited antigen from M. hyorhinis comprising the steps of:

extracting protein from cells of M. hyorhinis;

separating from the the cells a regression-associated antigen encoded by a purified and isolated nucleic acid described by a nucleotide sequence selected from the group consisting of:

the nucleotide sequence as shown in Fig. 4;

a nucleotide sequence which encodes the same sequence of amino acids as encoded by the nucleotide sequence shown in FIG. 4;

a nucleotide sequence which hybridizes with any 20 sequential nucleotides as shown in the nucleotide sequence shown in FIG. 4;

a nucleotide sequence which would hybridize with any 20 sequential nucleotides as shown in the nucleotide sequence shown in FIG. 4 but for the redundancy of the genetic code;

a nucleotide sequence which encodes an epitope encoded by 18 sequential nucleotides in the nucleotide sequence shown in FIG. 4; and

a purified and isolated nucleic acid described by a restriction map shown in FIG. 5.

43. An immortalized cell line producing a monoclonal antibody to a regression-associated antigen.

44. The immortalized cell line as recited in claim 43 wherein said cell line is the cell line designated as ATCC Deposit No. HB 9540.

45. The immortalized cell line as recited in claim 43 wherein said cell line secretes an $I_gM$ antibody to a regression-associated antigen.

46. A monoclonal antibody secreted by the cell line designated ATCC Deposit No. HB 9540.

47. An immunoassay kit comprising a regression-associated antibody selected from the group consisting of:

the regression-associated antibody described in claim 11;

the regression-associated antibody described in claim 45;

the regression-associated antibody described in claim 46;

48. The immunoassay kit recited in claim 47 further comprising a regression associated antibody selected from the group consisting of:

a neoplastic cell or membrane fractions thereof or regression-associated antigens isolated therefrom;

the regression-associated antigen as described in claim 11;

the regression-associated antigen described in claim 17;

the regression-associated antigen described in claim 19;

the regression-associated antigen described in claim 20;

the regression-associated antigen described in claim 22;

the regression-associated antigen described in claim 26; and

the regression-associated antigen described in claim 55.

49. An immunoassay kit comprising:

a regression associated antibody selected from the group consisting of:

a neoplastic cell or membrane fractions thereof or regression-associated antigens isolated therefrom;

the regression-associated antigen described in claim 6;

the regression-associated antigen described in claim 17;

the regression-associated antigen described in claim 19;

the regression-associated antigen described in claim 20;

the regression-associated antigen described in claim 22;

the regression-associated antigen described in claim 26; and

the regression-associated antigen described in claim 55.

50. A method of passive immunization comprising the step of injection into a patient an antibody selected from the group consisting of:

the antibody as described in claim 11;

the antibody as described in claim 45; and

the antibody as described in claim 46.

51. A method for immunotherapy of tumors comprising the steps of:

obtaining a first sample of serum from a patient diagnosed as not being in a state of regression and obtaining a second sample from a patient after diagnosis as being in a state of regression;

separately exposing a component of a neoplastic cell to the first and second samples;

determining the absence of immune complex formation between the component and antibodies in the first serum sample;

determining the presence of immune complex formation between an antibody and the component; and introducing into lymphatic or hematic fluid within a patient regression-associated antigens selected from the group consisting of:

a neoplastic cell or membrane fractions thereof or regression-associated antigens isolated therefrom;

the regression-associated antigen described in claim 6;

the regression-associated antigen described in claim 17;

the regression-associated antigen described in claim 19;

the regression-associated antigen described in claim 20;

the regression-associated antigen described in claim 22;

the regression-associated antigen described in claim 26; and

the regression-associated antigen described in claim 55.

52. An immunotherapeutic composition comprising:

a purified and isolated regression-associated antigen selected from the group consisting of:

a pharmaceutically acceptable diluent, adjuvant or carrier.

53. An immunotherapeutic composition comprising:

a purified and isolated regression-associated antibody selected from the group consisting of:

a pharmaceutically acceptable diluent, adjuvant or carrier.

the antibody as described in claim 11;

the antibody as described in claim 45; and

the antibody as described in claim 46.

54. A regression-associated antigen encoded by a purified and isolated nucleic acid described by a nucleotide sequence selected from the group consisting of:

a nucleotide sequence which encodes the sequence of amino acids Ser-Gly-Glu-Thr-Asp-Lys-Glu-Gly-Lys-Ile-Arg-Phe-Asp-Asn-X-Phe-Val-Lys-Asp wherein X may be Cys or Ser;

a nucleotide sequence which would hybridize with any 20 sequential nucleotides encoding Ser-Gly-Glu-Thr-Asp-Lys-Glu-Gly-Lys-Ile-Arg-Phe-Asp-Asn-X-Phe-Val-Lys-Asp wherein X may be Cys or Ser but for the redundancy of the genetic code; and

a nucleotide sequence which hybridizes with any 20 sequential nucleotides as shown in the nucleotide sequence encoding Ser-Gly-Glu-Thr-Asp-Lys-Glu-Gly-Lys-Ile-Arg-Phe-Asp-Asn-X-Phe-Val-Lys-Asp wherein X may be Cys or Ser; and

a nucleotide sequence which encodes an epitope encoded by 18 sequential nucleotides in the nucleotide sequence encoding Ser-Gly-Glu-Thr-Asp-Lys-Glu-Gly-Lys-Ile-Arg-Phe-Asp-Asn-X-Phe-Val-Lys-Asp wherein X may be Cys or Ser.

55. A regression-associated antigen encoded by a purified and isolated nucleic acid described by a nucleotide sequence selected from the group consisting of:

the nucleotide sequence as shown in FIG. 6;

a nucleotide sequence which encodes the same sequence of amino acids as encoded by the nucleotide sequence shown in FIG. 6;

a nucleotide sequence which would hybridize with any 20 sequential nucleotides as shown in the nucleotide sequence shown in FIG. 6;

a nucleotide sequence which would hybridize with any 20 sequential nucleotides as shown in the nucleotide sequence shown in FIG. 6 but for the redundancy of the genetic code; and

a nucleotide sequence which encodes an epitope encoded by 18 sequential nucleotides in the nucleotide sequence shown in FIG. 6.

56. A purified and isolated nucleic acid described by a nucleotide sequence selected from the group consisting of:

the nucleotide sequence as shown in FIG. 6;

a nucleotide sequence which encodes the same sequence of amino acids as encoded by the nucleotide sequence shown in FIG. 6;

a nucleotide sequence which hybridizes with any 20 sequential nucleotides as shown in the nucleotide sequence shown in FIG. 6;

a nucleotide sequence which would hybridize with any 20 sequential nucleotides as shown in the nucleotide sequence shown in FIG. 6 but for the redundancy of the genetic code; and

a nucleotide sequence which encodes an epitope encoded by 18 sequential nucleotides in the nucleotide sequence shown in FIG. 6.

57. A cell transformed with a nucleic acid as recited in claim 56.

58. An expression product of a cell as recited in claim 57.

59. A monoclonal or monospecific polyclonal antibody exhibiting a specific immunoreactivity with an antigen as recited in claim 55.

60. A monoclonal or monospecific polyclonal antibody exhibiting a specific immunoreactivity with an expression product as recited in claim 58.

61. A method for isolating a regression-associated antigen from M. hyorhinis comprising the steps of:

extracting protein from cells of M. hyorhinis;

separating from the the cells a regression-associated antigen encoded by a purified and isolated nucleic acid described by a nucleotide sequence selected from the group consisting of:

the nucleotide sequence as shown in FIG. 6;

a nucleotide sequence which encodes the same sequence of amino acids as encoded by the nucleotide sequence shown in FIG. 6;

a nucleotide sequence which hybridizes with any 20 sequential nucleotides as shown in the nucleotide sequence shown in FIG. 6;

a nucleotide sequence which would hybridize with any 20 sequential nucleotides as shown in the nucleotide sequence shown in FIG. 6 but for the redundancy of the genetic code; and

a nucleotide sequence which encodes an epitope encoded by 18 sequential nucleotides in the nucleotide sequence shown in FIG. 6.

62. A purified and isolated nucleic acid described by a restriction map shown in FIG. 7.

63. A cell transformed with a nucleic acid recited in claim 62.

64. An expression product of a cell as recited in claim 62.

41

# RAAs DETECTED IN MEMBRANES OR MEDIA AFTER
# I.L.( Pt-1) OR S.C.( Pt-2, rabbit 209) IMMUNIZATIONS

FIG.1

FIG.2

COMPARISON OF
TWO VACCINE LYSATES

FIG.3

FIG. 4/1

FIG. 4

THE NUCLEOTIDE SEQUENCES AND THE DEDUCED

NUCLEOTIDE AMINO ACIDS SEQUENCE OF 38 kd RAA

GCGTTATTAT AAAAAATAAA AATTTAAAAA GTAATGTAAA AATTTAATTT ACATTACTTT TTTTGTATAA TTATTTCAAC

AGGGGTGCTG TAAAAGGTTG AGAAATACTC TATAAGTTGA TCTAGATAAT GCTAGCGTAA CGAGTTGTTT TTTATTTTCA

                                                                    *    *        *
                                                              leu leu lys lys phe lys asn phe
AATTTTTAAA GCTATCTCTG TCACAAAAAT TAATTAACGG AGGTAGCTTT TTTG CTC AAA AAA TTT AAA AAT TTT

ile leu phe ser ser ile phe ser pro ile ala phe ala ile ser cys ser asn thr gly val val
ATT CTA TTT TCA TCT ATA TTT TCG CCA ATA GCA TTT GCT ATA TCA TGT TCT[AAT ACA GGA GTA GTC

*        *    *                                    *    *                              *
lys gln glu asp  val ser val ser gln gly gln trp asp lys ser ile thr phe gly val ser glu
AAG CAA GAG GA]T GTA TCA GTT AGT CAA GGT CAA TGA GAT AAA AGT ATA ACA TTT GGT GTT TCA GAA

          *    *    *        *    *        *    *                          *
ala trp leu asn lys lys lys gly gly lys glu val asn lys glu val ile asn thr phe leu glu
GCT TGG TTA AAC AAG AAA AAA GGA GGT AAA GAA GTT AAC AAA GAA GTT ATT AAT ACA TTT TTA GAA
HindIII                                            HpaI

FIG. 4/2

```
        *    *    *              *         *                   *    *.        *              *    *
asn  phe  lys  lys  glu  phe  asn  lys  leu  lys  asn  ala  asn  asp  lys  thr  lys  asn  phe  asp  asp  val
AAT  TTC  AAA  AAA  GAA  TTT  AAT  AAA  CTC  AAA  AAT  GCA  AAT  GAT  AAA  ACC  AAA  AAC  TTC  GAT  GAC  GTT

  *         *                        *                                   _____              *
asp  phe  lys  val  thr  pro  ile  gln  asp  phe  thr  val  leu  leu  asn  asn  leu  ser  thr  asp  asn  pro
GAT  TTT  AAA  GTA  ACT  CCA  ATT  CAA  GAC  TTT  ACT  GTG  TTG  TTA  AAC  AAT  TTA  TCT  ACT  GAC  AAT  CCT

  *         *              _____              *         *         *
glu  leu  asp  phe  gly  ile  asn  ala  ser  gly  lys  leu  val  glu  phe  leu  lys  asn  asn  pro  gly  ile
GAA  TTA  GAT  TTT  GGA  ATT  AAT  GCT  TCA  GGA  AAA  TTG  GTA  GAA  TTT  CTA  AAA  AAT  AAT  CCT  GGT  ATA

                 *                                         *    *    *    *    *    *
ile  thr  pro  ala  leu  glu  thr  thr  thr  asn  ser  phe  val  phe  asp  lys  glu  lys  asp  lys  phe  tyr
ATA  ACT  CCA  GCA  TTA  GAA  ACA  ACA  ACT  AAT  TCT  TTT  GTA  TTT  GAC  AAA  GAA  AAA  GAT  AAA  TTT  TAT

    *              *         *              *         *    *         *                   *
val  asp  gly  thr  asp  ser  asp  pro  leu  val  lys  ile  ala  lys  glu  ile  asn  lys  ile  phe  val  glu
GTT  GAT  GGT  ACA  GAT  TCA  GAT  CCA  CTT  GTA  AAA  ATT  GCT  AAA  GAA  ATT  AAT  AAA  ATT  TTT  GTT  GAA

                      *    *              *
thr  pro  tyr  ala  ser(trp)thr  asp  glu  asn  his  lys(trp)asn  gly  asn  val  tyr  gln  ser  val  tyr
ACT  CCA  TAT  GCA  AGT  TGA  ACT  GAT  GAA  AAT  CAT  AAG  TGA  AAT  GGT  AAT  GTT  TAT  CAA  AGT  GTT  TAC
```

EP 0 308 265 A2

FIG. 4/3

```
 *                                           *                         *               *   *
asp pro thr val gln ala asn phe tyr arg gly met ile(trp)ile lys gly asn asp glu thr leu
GAT CCA ACT GTT CAA GCT AAT TTT TAT AGA GGA ATG ATT TGA ATA AAA GGT AAT GAT GAA ACT CTA
                                                                                     AluI


      *           *   *                 *   *   *                 *
ala lys ile lys lys ala(trp)asn asp lys asp(trp)asn thr phe arg asn phe gly ile leu his
GCT AAA ATT AAA AAA GCT TGA AAT GAT AAA GAT TGA AAT ACA TTT AGA AAT TTT GGA ATT TTA CAC
            AluI-HindIII


      *   *   _____       *       *       *   *               *
gly lys asp asn ser ser ser lys phe lys leu glu glu thr ile leu lys asn his phe gln asn
GGT AAA GAT AAT TCT TCT TCT AAA TTC AAG TTA GAA GAA ACT ATA TTA AAA AAC CAC TTT CAA AAT
            MboII/MboII


 *               *   *   *                         *       *           *
lys phe thr thr leu asn glu asp arg ser ala his pro asn ala tyr lys gln lys ser ala asp
AAA TTT ACA ACA CTA AAT GAA GAC AGA AGC GCA CAT CCA AAC GCA TAT AAA CAA AAA TCT GCA GAT
            MboII         HhaI                                              PstI


          *   *                       *   *
thr leu gly thr leu asp asp phe his ile ala phe ser glu glu gly ser phe ala(trp)thr his
ACA TTG GGA ACT TTA GAT GAT TTC CAT ATT GCT TTT TCA GAA GAA GGT TCT TTT GCT TGA ACA CAT
                                                            MboII
```

EP 0 308 265 A2

FIG. 4/4

```
        *                  *             *        *              *    *          *
asn lys ser ala thr lys pro phe glu thr lys ala asn glu lys met glu ala leu ile val thr
AAC AAA TCA GCA ACA AAA CCT TTT GAA ACT AAA GCA AAT GAA AAG ATG GAA GCA CTT ATA GTA ACT

                   *                          *    *                        *
asn pro ile pro tyr asp val gly val phe arg lys ser val asn gln leu glu gln asn leu ile
AAT CCA ATT CCG[TAT GAT GTT GGA GTG TTT]AGA AAA AGT GTT AAC CAA TTA GAA CAA AAT TTA ATT
                                                         HpaI

                        *           *         *
val gln thr phe ile asn leu ala lys asn lys gln asp thr tyr gly pro leu leu gly tyr asn
GTT CAA ACA TTC ATT AAT TTA GCT AAA AAT AAA CAA GAT ACA TAT GGT CCA CTT TTA GGG TAT AAT
                        AluI

        *    *         *              *    *           *            *    *              *
gly tyr lys lys ile asp asn phe gln lys glu ile val glu val tyr glu lys ala ile lys end
GGT TAT AAA AAA ATT GAC AAT TTC CAA AAA GAG ATT GTA GAA GTT TAT GAA AAA GCC ATT AAA TAA

ATT AGA AAT AAA AAA TTT AAC ATT TAA AAA TAA AAA TGA TGA TTA CAT AAT TTT AAA GAA CCT AAA

CTT AGA TAT AAA TTC TGA TAA AGT TTT GTT TTT ATT AGG TTC ATC CAG GCC
                                                            HaeIII
```

EP 0 308 265 A2

FIG. 5A

Hae II  Pvu II    Ssp I  Rsa I  Hinf I  Hind III  Cfo I  Alu I  Hae III  Ssp I  Hinf I  Rsa I    Cfo I  Hind III    M. hyorhinis DNA

→ 38kDa

—— 1 kb

FIG. 5B

Hind III  Hae III    Hind III    pM38- 29/3

FIG. 5C

Rsa I  Hinf I  Hae III  Hinf I  Rsa I    pM38-EI-I

EP 0 308 265 A2

FIG. 6/1

FIG. 6

The Nucleotide Sequence and the Deduced Amino Acid Sequence of 43 kd RAA

CTACAGTTATA GGTAATGGAA GTGGAATTTT CGAAAGTATT AAAACTTATC AAAATATTTT CTATTTATTA CCATATTTAG
                    TaqI                                SspI

TAACATTAAT AATATTAATA TTTACATCAA AAAATACAAT TGCTCCAAAA GCAGTCGGTC TTCCTTATGA CAAATCATTG
       SspI    SspI

AGGTAGAAGA TCAAAAACTT TATATTTTTT TTGTTTTTAT GGTATATTAT GTTTTAAAT TGTACATTTA ATTATTAAGT GAGGTGAATT T
                                                   RsaI

                              Met Asn Phe Lys Lys Ser Leu Leu Phe Leu Thr Gly Thr Ile
                              ATG AAT TTT AAA AAA TCA TTA TTA TTT TTA ACA GGA ACA ATA
                                      45                  60

                            mature N-terminus
Ser Thr Val Ala Ser Val Ala Thr Phe Val Ser Cys Gly Glu Thr Asp Lys Glu Gly Lys Ile Ile Arg Ile
TCA ACA GTA GCA TCA GTA GCA ACC TTT GTT TCT TGT GGA GAA ACT GAC AAA GAA GGA AAA ATA ATA AGA ATT
 75                90               105             120             135

Phe Asp Asn Ser Phe Val Lys Asp Arg Gln Ala Glu Ile Glu Lys Ala Lys Asn Phe Asp Phe Asn Thr Val
TTT GAT AAT TCT TTT GTT AAA GAT AGA CAA GCA GAA ATA GAA AAA GCA AAA AAC TTT GAC TTT AAC ACA GTT
  150             165             180            195            210

EP 0 308 265 A2

FIG. 6/2

Leu Leu Thr Ala Gly Gly Thr Val Gln Asp Lys Ser Phe Asn Gln Ser Ile(trp)Glu Ala Val Leu Glu His
TTA TTA ACA GCA GGC GGA ACT GTA CAA GAC AAA TCA TTT AAT CAA TCA ATT TGA GAA GCT GTT TTA GAG CAT
        225                     240                     255                     270                     285
                    RsaI

Tyr Asp Gln Ile Glu Lys Thr Thr Asn Leu Asp Arg Val Ser Gln Glu Thr Asn Asn Gln Ser Glu Leu Ile
TAT GAT CAA ATA GAA AAA ACA ACT AAT CTT GAT AGA GTT TCA CAA GAG ACT AAT AAT CAA TCT GAA CTT ATT
        300                     315                     330                     345                     360
  BclI/Sau3A

Gly Lys Tyr Lys Asn Phe Leu Asn Gly Asn Lys Asn Val(trp)Ile Leu Thr Gly Phe Gln Gln Gly Gln Glu
GGT AAG TAC AAA AAT TTT TTA AAT GGA AAT AAA AAT GTT TGA ATT TTA ACC GGT TTT CAA CAA GGA CAA GAA
        375                     390                     405                     420
    RsaI                                                                    HpaII

Phe Pro Lys Phe Leu Lys Gln Thr Asp Ser Asn Gly Lys Lys Tyr Ser Asp Leu Leu Ala Glu Lys Lys Val
TTT CCA AAG TTT TTA AAA CAA ACC GAT TCT AAT GGT AAA AAA TAC AGC GAT TTA TTA GCA GAA AAA AAA GTC
435                     450                     465                     480                     495
                    HinfI

Ile Ile Val Ala Val Asp(trp)Asp Leu Ser Lys Glu Asp Lys Asp Leu Ile Lys Ala Gly His Phe Ile Ser
ATA ATA GTT GCA GTA GAT TGA GAT TTA TCA AAA GAA GAT AAA GAT TTA ATT AAA GCA GGA CAC TTT ATT TCA
        510                     525                     540                     555                     570

FIG. 6/3

```
Leu Leu Tyr Lys Thr Glu Glu Ala Gly Phe Ile Ala Gly Tyr Ala Ser Ser Lys Phe Leu Ala Tyr Lys Phe
TTA CTA TAT AAA ACA GAA GAA GCA GGT TTT ATT GCA GGG TAT GCG TCG TCT AAA TTT TTA GCA TAT AAA TTC
        585             600             615             630             645

Pro Asn Asp Glu Ala Lys Arg Thr Ile Ala Pro Phe Gly Gly Gly His Gly Ala Gly Val Thr Asp Phe Ile
CCA AAT GAT GAA GCA AAA AGA ACT ATT GCT CCA TTT GGT GGT GGA CAC GGA GCT GGA GTT ACT GAC TTT ATA
        660             675             690             705             720

Ala Gly Phe Leu Ala Gly Ile Ala Lys Tyr Asn Asn Asp Asn Pro Thr Ala Lys Val Thr Ile Ser Asp Asn
GCG GGA TTT TTA GCA GGA ATA GCA AAG TAT AAC AAT GAC AAT CCA ACC GCT AAA GTA ACA ATT TCA GAT AAT
            735             750             765             780

Asn Ile Asn Ile Asp Thr Gly Phe Ile Ser Asn Asp Lys Thr Ala Thr Phe Ile Asn Gly Ile Val Asn Lys
AAT ATT AAC ATT GAT ACA GGT TTT ATT TCT AAT GAT AAA ACA GCT ACA TTT ATT AAT GGA ATA GTA AAT AAA
795             810             825             840             855

Ser Ser Leu Val Leu Pro Val Val Gly Ser Leu Thr Ser Ser Val Val Asp Ala Ile Lys Lys Ser Asn Lys
TCT TCA CTT GTT TTA CCA GTG GTA GGT TCA TTA ACT AGC TCA GTG GTT GAT GCA ATA AAA AAA TCA AAT AAA
    870             885             900             915             930
                                                        DdeI

Asp Thr Lys Tyr Leu Ile Gly Val Asp Thr Asp Gln Ser Lys Ile Phe Pro Pro Ala Thr Val Phe Phe Thr
GAC ACA AAA TAC TTA ATA GGT GTA GAC ACA GAT CAA TCA AAA ATT TTT CCC CCT GCT ACA GTC TTT TTC ACA
    945             960             975             990             1005
    AccI            Sau3A
```

FIG. 6/4

Ser Ile Glu Lys His Leu Gly Arg Thr Ile Tyr Glu Val Leu Thr Asp Ile(trp)Leu Lys Lys Glu Asp Ser
TCA ATA GAA AAA CAT TTA GGA AGA ACC ATT TAC GAA GTC TTA ACT GAT ATT TGA CTA AAA AAA GAA GAT TCT
        1020                    1035                1050                1065                1080
                                                                                            HinfI


Lys Phe Leu Gly Ser Phe Arg Ser Phe Lys Leu Thr Asn Pro Ala Asn Ala Thr Val Tyr Lys Gly Ile Ser
AAA TTT TTA GGT TCA TTT AGA TCA TTC AAG TTA ACA AAT CCA GCA AAC GCT ACA GTT TAT AAA GGA ATT TCA
            1095                    1110                1125                1140
            Sau3A        HpaI/HincII


Asp Asp Phe Val Gly Val Ser Asn Ser Thr Val Ala Asp Ala Asp Lys Val Lys Ala Gln Glu Phe Leu Asn
GAT GAT TTC GTT GGT GTT TCT AAT TCT ACA GTT GCA GAT GCA GAT AAA GTA AAA GCA CAA GAA TTT TTA AAT
1155            1170                1185                1200                1215


Glu Ala Thr Ala Asp Phe Lys Lys Gln Ile Gln Ala Asn Pro Thr Asn Tyr Lys Ser Val Leu Gly Ile Pro
GAA GCT ACA GCA GAT TTT AAA AAA CAA ATT CAA GCC AAT CCA ACA AAT TAC AAA AGT GTT TTA GGT ATT CCT
    1230                1240                1260                1275                1290
    RsaI


Thr Met Leu Ile Asn Asp Asn Asp Ala Lys Asp Asn Glu Lys Ala Leu Asn Glu Leu Ile Lys Lys Ile Asn
ACA ATG TTA ATT AAT GAT AAT GAT GCA AAA GAT AAT GAA AAA GCT TTA AAC GAA TTA ATT AAA AAA ATT AAC
        1305                1320                1335                1350                1365
                                        HindIII

FIG. 6/5

Gly Thr Thr Thr Thr Thr Ala ...3' untranslated
GGA ACA ACT ACT ACA ACA GCA TAA TAT CTA TTC ATA GAT AGA TAG AAA TTC AAC AAT AAT TTA AAA ACA ACA
         1380                    1395                 1410                 1425                 1440


CCC AAA TCA AGG TGT TGT TTT TAA TTC GAA ATA TTA CAA AAA TAA TAT AAT TAT TTA ATA TCT CAA TAG CAA
             1455                1470             1485                 1500
                        AsuII/TaqI


TAA TAG CAG AAT ATA ATC CTT TTC ATA ATG GTC ATA TTT ATC AAC TAA ATT ATG TTA AAA AAC ATT TTC CAA
1515                 1530                 1545                 1560                 1575


ATG AAA AAA TTG TTG TAA TCT TAA GCG GAA AAT ATA CTC AAA GAG GTG AGT TGG CAG TAG CTG ATT TTG AAA
     1590                 1605                 1620                 1635                 1650


CTA GAA AAC AAT TTG CTT TAA AAT TTG GAG CAG ATG AAG TTA TAA ATT ACT TTA ATA TGC A
         1665                 1670                 1695                 1710

EP 0 308 265 A2

FIG. 7A

Hind III  EcoR I  Pst I  Hpa I  Hind III  Sco I  Xba I  EcoRI  Hind III

M. hyorhinis DNA

→ 43 kDa

FIG. 7B

pM43-8

FIG. 7C

pM43-1

1kb

EP 0 308 265 A2